# FASCICULE DE BREVET EUROPEEN

(11) **EP 3 555 112 B1**
(45) Date de publication et mention de la délivrance du brevet: **06.12.2023**
(21) Numéro de dépôt: 17821597.6
(22) Date de dépôt: 19.12.2017
(51) Int. Cl.: C07H 15/20, C07H 17/02, C12Q 1/34, G01N 33/574, G01N 33/68, A61K 49/00

(54) **SUBSTRAT DE GLYCOSIDASE FLUOROGENE ET PROCEDE DE DETECTION ASSOCIE**
FLUOROGENES GLYCOSIDASE-SUBSTRAT UND ZUGEHÖRIGES DETEKTIONSVERFAHREN
FLUOROGENIC GLYCOSIDASE SUBSTRATE AND ASSOCIATED DETECTION METHOD

(30) Priorité: 19.12.2016 FR 1662787
(43) Date de publication de la demande: 23.10.2019
(73) Titulaire: Ecole Normale Supérieure de Lyon, 69007 Lyon (FR); Université Claude Bernard Lyon 1, 69100 Villeurbanne (FR); Centre National de la Recherche Scientifique, 75016 Paris (FR)
(72) Inventeur: HASSERODT, Jens, 69110 Sainte-Foy-Les-Lyon (FR); GONDRAND, Corentin, 69117 Heidelberg (DE); PROST, Maxime, 69007 Lyon (FR); YVERT, Gaël, 69230 Saint-Genis-Laval (FR); TRIQUENEAUX, Gérard, 69006 Lyon (FR)
(74) Mandataire: Cabinet Becker et Associés
(86) Numéro de dépôt international: PCT/EP2017/083518
(87) Numéro de publication internationale: WO 2018/114933

(56) Documents cités:
- EP-A1- 2 033 663
- WO-A1-2013/045854
- WO-A1-2014/020285
- FENGTIAN XUE ET AL: "Kinetic delay of cyclization/elimination-coupled enzyme assays: Analysis and solution", BIOORGANIC & MEDICINAL CHEMISTRY LETTERS, vol. 21, no. 3, 1 février 2011 (2011-02-01), pages 1069-1071, XP055042989, ISSN: 0960-894X, DOI: 10.1016/j.bmcl.2010.09.104
- GROOT DE FRANCISCUS M H ET AL: "Elongated Multiple Electronic Cascade and Cyclization Spacer Systems in Activatible Anticancer Prodrugs for Enhanced Drug Release", THE JOURNAL OF ORGANIC CHEMISTRY, AMERICAN CHEMICAL SOCIETY ETC.|, vol. 66, 1 janvier 2001 (2001-01-01), pages 8815-8830, XP002212035, ISSN: 0022-3263, DOI: 10.1021/JO0158884

## Description

La présente invention concerne les sondes pour détecter l'activité enzymatique de type glycosidase. En particulier, l'invention concerne de nouveaux substrats fluorogènes pour détecter la présence d'une glycosidase catalytiquement active et un procédé de détection utilisant de tels substrats.

Dans l'analyse d'un échantillon biologique ou chimique, la détection d'une activité glycosidase peut être très utile (Boonacker E. et Van Noorden C. J. F. (2001). Enzyme cytochemical techniques for metabolic mapping in living cells, with special référence to proteolysis. J. Histochem. Cytochem. 49, 1473-1486 ; Perry, J. D., James, A. L., Morris, K. A., Oliver, M., Chilvers, K. F., Reed, R. H., & Gould, F. K. (2006). Evaluation of novel fluorogenic substrates for the détection of glycosidases in Escherichia coli and enterococci. Journal of Applied Microbiology, 101(5), 977-985 ; Orenga, S., James, A. L., Manafi, M., Perry, J. D., & Pincus, D. H. (2009). Enzymatic substrates in microbiology. Journal of Microbiological Methods, 79(2), 139-155). Les organismes entiers, les cellules ou les extraits cellulaires, les liquides biologiques ou les mélanges chimiques sont des exemples d'échantillons biologiques ou chimiques dans lesquels une activité glycosidase peut être détectée. Les glycosidases sont une vaste famille d'enzymes qui inclut de nombreux biomarqueurs de diverses pathologies. Elles sont impliquées aussi dans de nombreux processus cellulaires bénins et font donc l'objet d'études innombrables de la part des biologistes cellulaires. Ainsi, leur détection peut donner des informations concernant un état métabolique ou morbide particulier, par exemple. Une détection efficace permettrait également de mettre en place des cribles à haut débit, offrant la possibilité de détecter de nouvelles glycosidases naturelles, ou de développer de nouvelles glycosidases par évolution dirigée d'enzymes connues, ou d'améliorer les performances glycolytiques de certains microorganismes par mutagénèse ou évolution expérimentale de leur génome.

De ce fait, une sonde capable de détecter une activité glycosidase est très utile.

La détection de cette activité par capture de lumière de fluorescence émise par une sonde est un procédé bien plus sensible que la collecte du reste de lumière blanche lors d'une simple absorption par la sonde, c'est-à-dire que le seuil de détection est bien plus bas. La détection d'une émission de fluorescence est très aisée à mettre en oeuvre, de sorte que les sondes fluorescentes sont des outils très attractifs pour les sciences de la vie. Par exemple, la classe des fluorophores conduisant à un transfert de proton intramoléculaire dans un état excité, nommés ESIPT (ESIPT de l'anglais « Excited State Intramolecular Proton Transfer »), est décrite, notamment, dans a) Ormson, S.M., et al. Progress in Reaction Kinetics (1994) 19, 45-91 ; b) Legourrierec, D., et al. Progress in Reaction Kinetics (1994), 19, 211-275 ; et c) Zhao, J., Ji, S., Chen, Y., Guo, H., & Yang, P. (2012). Excited state intramolecular proton transfer (ESIPT): from principal photophysics to the development of new chromophores and applications in fluorescent molecular probes and luminescent materials. Physical Chemistry Chemical Physics, 14 (25), 8803. La première interprétation de la fluorescence élevée trouvée dans certains composés phénoliques comme étant un phénomène ESIPT peut être attribuée à Weller (pour le salicylate de méthyle : Weller, A. (1961). Fast Reactions of Excited Molecules. Progress in Reaction Kinetics and Mechanism 1, 187), et à Heller et Williams (pour les hydroxyphenylbenzoxazoles : Heller A., et Williams, D.L., J. Phys. Chem. (1970) 74, 4473-4480).

La classe des fluorophores ESIPT est particulièrement attractive pour le chercheur dans les sciences de la vie, du fait de ses propriétés exceptionnelles comparées aux fluorophores conventionnels. Les propriétés exceptionnelles des fluorophores ESIPT sont :
(a) un grand déplacement de Stokes dépassant souvent 130 nm et capable d'atteindre des valeurs de 250 nm qui permet des choix instrumentaux maximisant la sensibilité de détection ;
(b) une excellente résistance au photoblanchiment avec des taux qui peuvent être de plusieurs ordres de grandeur supérieurs à ceux de fluorophores modèles comme la fluorescéine ;
(c) la possibilité de concevoir des fluorophores qui émettent une fluorescence brillante à l'état solide, une propriété rare parmi tous les fluorophores connus. Cette dernière performance permet la production d'un signal de haute intensité au niveau du site d'activation, avec une dilution minimale causée par la diffusion;
(d) la possibilité de concevoir des fluorophores ESIPT qui émettent dans le rouge ou le proche infrarouge (600 à 850 nm) où la transparence des tissus est la plus élevée ; une sonde utilisant de tels fluorophores serait alors particulièrement appropriée pour l'imagerie chez l'animal vivant ; et enfin
(e) la possibilité de concevoir un substrat n'émettant pas de fluorescence en remplaçant l'atome d'hydrogène porté par l'hydroxyle d'un fluorophore ESIPT par un substituant possédant une réactivité spécifique vis-à-vis d'un analyte chimique ou biochimique, le clivage de ce substituant entraînant l'apparition de la fluorescence.

Le niveau de sensibilité d'une méthode de détection de l'activité enzymatique, par utilisation d'un substrat donnant lieu à une production de fluorescence, est étroitement lié (i) au taux de photoblanchiment, (ii) au degré d'accumulation du signal fluorescent sur son site de production (et donc au taux de diffusion depuis ce site, et à la question de savoir si le fluorophore précipite ou non) (iii) au mode éteint/allumé réel dans lequel le substrat fonctionne (absence de bruit de fond qui serait dû à une fluorescence du substrat non transformé), et (iv) au degré de superposition du spectre d'excitation et du spectre d'émission (leur séparation au niveau de la ligne de base étant la configuration la plus favorable ; voir point (a) ci-dessus). Le point (iv) revêt une importance toute particulière, car la séparation complète au niveau de la ligne de base offre l'opportunité d'un choix de filtres très large pour la source lumineuse (pour exciter le fluorophore à toutes les longueurs d'ondes possibles), mais de manière encore plus importante, pour le détecteur (pour récolter des photons provenant de toutes les longueurs d'ondes émises par le fluorophore). Le point (iv) minimise aussi la perturbation du processus de détection par l'autofluorescence tissulaire (caractérisée par un faible déplacement de Stokes des fluorophores naturels), un problème récurrent rencontré avec les fluorophores établis, qui présentent eux aussi un faible déplacement de Stokes.

Parmi la classe importante des fluorophores ESIPT, la dichloro-HPQ (6-chloro-2-(5-chloro-2-hydroxyphenyl)-4(3H)-quinazolinone ; numéro CAS : 28683-92-3) est particulièrement intéressante, étant donné qu'elle est parfaitement insoluble dans les milieux aqueux/physiologiques, tout en étant fortement fluorescente à l'état solide et seulement à l'état solide. Néanmoins, il est très difficile d'utiliser la dichloro-HPQ dans la conception d'une sonde moléculaire qui renseigne sur l'activité d'une glycosidase. D'ailleurs, les principales activités pour lesquelles une sonde à base de HPQ a déjà été conçue (et commercialisée) sont celles des phosphatases, du fait de l'impossibilité de créer une sonde à base de HPQ stable avec un hydroxyle phénolique glycosylé car le produit résultant est enclin à une hydrolyse spontanée rapide qui, bien entendu, libère la dichloro-HPQ insoluble libre et produit ainsi un signal fluorescent erroné ("signal de fond"). Il convient également de noter que la commercialisation par Molecular Probes de tels composés glycosylés (ELF 97 substrat glucuronidase (No. E6587) et ELF 97 substrat chitinase/N-acetylglucosaminidase (No. E22011) a été interrompue en 2008 en raison de l'instabilité à l'hydrolyse intrinsèque des glycosides phénoliques, et en particulier de ceux qui sont construits à partir des phénols appauvris en électrons, telle que la dichloro-HPQ. En effet, il est connu que n'importe quel glycoside à base d'un nitrophénol (qui est un phénol aussi appauvri en électrons que la dichloro-HPQ) s'hydrolyse spontanément à pH physiologique. Il est également connu que ce problème de stabilité s'aggrave sévèrement à des pH plus acides (par exemple à un pH de 6,5), par comparaison au pH physiologique (pH 7,4). Au cours des dernières années, il y a eu un intérêt croissant dans la conception de substrats d'enzymes à plusieurs composants déclencheur/espaceur(s)/fluorophore en utilisant des espaceurs à auto-immolation comme agent de liaison. On peut notamment citer les travaux de l'un des inventeurs de la présente demande de brevet correspondant aux demandes WO 2013/045854, WO 2014/020285 et WO 2015/197981 qui utilisent un espaceur auto-effondrable reliant un substrat de peptidase et/ou glycosidase à un groupe aryle entraînant, après clivage du substrat, la cyclisation de l'espaceur et la libération d'un fluorophore ESIPT.

Le document EP2033663 décrit des substrats fluorescents de nature saccharidique comprenant un bras espaceur autosécable fonctionalisé par un fluorophore F et par un inhibiteur de la fluorescence de F. Ces substrats peuvent être utilisés pour la détection d'activités enzymatiques in vivo.

Cependant, avec les technologies existantes, la détection des glycosidases n'est pas suffisamment fiable (les sondes sont instables et libèrent de la fluorescence en l'absence de l'enzyme cible), requiert un temps trop long (la réponse enzymatique est trop lente) et/ou n'est pas suffisamment précise (le fluorophore libéré ne précipite pas en un point mais diffuse dans le milieu).

Dans ce contexte, les Demandeurs proposent de nouveaux substrats d'enzyme glycosidase offrant une cinétique de réponse enzymatique particulièrement rapide. L'invention propose d'utiliser un nouvel espaceur qui permet de créer une sonde stable, adaptée à l'incorporation d'un fluorophore ESIPT, avec ainsi une minimisation de la fluorescence de fond de la sonde non transformée, et qui permet une augmentation significative de la sensibilité, et, de ce fait, pourrait conduire à une réduction de la quantité à utiliser et pourrait ainsi, notamment, rendre possible une application en imagerie *in vivo,* tout en réduisant les problèmes de toxicité. L'objectif de l'invention est de proposer de nouveaux substrats de glycosidase qui soient stables en milieu aqueux et qui restent non fluorescents ou faiblement fluorescents à une longueur d'onde bien différente de celle à laquelle le fluorophore libéré est lui fluorescent, mais qui réagissent rapidement avec des glycosidases pour produire une petite molécule fluorescente correspondant à un fluorophore ESIPT. Selon l'invention, il est proposé un substrat de glycosidase ayant les propriétés suivantes :
- une grande spécificité pour une glycosidase particulière, en fonction du choix du groupement glycosyle présent sur la sonde ;
- une absence de fluorescence de fond grâce à une haute stabilité de la sonde en l'absence de l'enzyme cible, une précipitation du fluorophore sur le site de l'enzyme et une absence de diffusion du fluorophore dans le milieu ; et
- une cinétique rapide de transformation sous l'action de la glycosidase cible.

Le substrat de glycosidase selon l'invention permet ainsi de conserver les avantages des substrats de glycosidase de l'art antérieur (i.e. grande spécificité pour une glycosidase particulière et absence de fluorescence de fond), tout en ayant une cinétique rapide de transformation sous l'action de la glycosidase cible.

Plus précisément, l'invention concerne des composés de formule (I) : 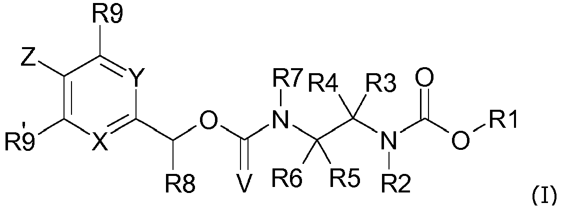 dans laquelle :
- R1 est tel que HOR1, obtenu après clivage de la liaison -C(O)-OR1 présente sur la formule (I), appartient à la classe des fluorophores conduisant à un transfert de proton intramoléculaire dans un état excité, nommés ESIPT, R1 étant un groupe aromatique avec -OR1 qui répond à la formule (A1) telle que définie dans la revendication 1 et telle que détaillée ci-après,
- R2, R3 et R4 sont définis comme suit :
   ∘ soit R2 est un (C1-C4)alkyle, R3 est un (C1-C4)alkyle ou un atome d'hydrogène, et R4 est un (C1-C4)alkyle,
   ∘ soit R3 est un (C1-C4)alkyle ou un atome d'hydrogène et R2 et R4 sont liés entre eux et forment avec les atomes de carbone et d'azote auxquels ils sont liés un hétérocycle aliphatique, cet hétérocycle pouvant être substitué par un groupement hydrosolubilisant,
   ∘ soit R2 est un (C1-C4)alkyle et R3 et R4 sont liés entre eux et forment avec l'atome de carbone auxquels ils sont liés un carbocycle aliphatique,
- R5 et R6 sont identiques ou différents et représentent indépendamment l'un de l'autre un atome d'hydrogène, un (C1-C4)alkyle, ou un (C5-C10)aryle,
- R7 est un atome d'hydrogène, ou un groupe choisi parmi les (C1-C4)alkyle et (C1-C4)alcoxy,
- R8 représente un atome d'hydrogène, ou un groupe (C1-C10)alkyle, substitué ou non substitué, ou un groupe -D1-D2-D3 avec :
   ∘ D1 représentant un groupe triazolyle ou -CH₂-triazolyle,
   ∘ D2 représentant un groupe (C1-C10)alkylène, (C1-C10)alcènylène, ou (C1-C10)alcynylène, lesdits groupes étant éventuellement interrompus par un ou plusieurs hétéroatomes choisis parmi O ou N, un groupe glycosyle divalent, un groupe -O-(CHR-CHR'-O)ₙ- ou -N-(CHR-CHR'-O)ₙ-, n étant un entier variant de 1 à 20, R et R', identiques ou différents, représentant H ou CH₃ à condition que R et R' ne soient pas simultanément CH₃, un acide aminé ou un peptide, ou une combinaison de ces groupes,
   ∘ D3 représentant un motif maléimidocaproyl, acide aminé, peptide, acide folique, anticorps ou fragment d'anticorps lié à D2, par une fonction acide carboxylique qu'il comprend, formant un lien ester ou amide,
- R9 et R'9, identiques ou différents, représentent un atome d'hydrogène, ou un groupe électro-attracteur tel qu'un atome d'halogène, ou un groupe choisi parmi - NO₂, -CN ou -NH-C(O)-CH₂-Ab avec Ab représentant un anticorps,
- V représente un atome d'oxygène ou un atome de soufre,
- X, Y et Z sont tels que :
   ∘ soit X représente CR10, Y représente CR'10 et Z représente OR0,
   ∘ soit X représente CR10, Y représente CORO et Z représente R'10,
   ∘ soit X représente CR10, Y représente un atome d'azote et Z représente OR0,
   ∘ soit X représente un atome d'azote, Y représente CORO et Z représente R10
      avec :
      ∘ R0 représentant un groupement glycosyle lié par son carbone anomérique au reste de la molécule de formule (I), et
      ∘ R10 et R'10, identiques ou différents, représentant un atome d'hydrogène ou un groupe électro-donneur tel qu'un (C1-C20)alkyle, un (C5-C24)aryle, ou un (C1-C20)alcoxy,
sous la forme d'un mélange d'isomères optiques selon toutes proportions, ou sous une forme enrichie en un isomère optique.

Les composés (I) selon l'invention, en fonction du groupe glycosyle sélectionné, agissent comme une sonde moléculaire capable de révéler la présence d'une activité enzymatique glycosidase spécifique par détection de fluorescence. La liaison R0-O présente dans les composés de formule (I) est susceptible d'être clivée, par hydrolyse, en présence d'une enzyme glycosidase agissant en tant que catalyseur de la réaction de clivage.

Plus spécifiquement, la sonde est invisible avant de rencontrer l'enzyme glycosidase ciblée, (c'est-à-dire "sonde furtive"), mais quand elle est modifiée chimiquement par ladite enzyme, elle se fragmente *via* une réaction en cascade pour produire une fluorescence intense. La sonde est composée de 4 composants moléculaires : i) un tandem d'espaceurs auto-effondrables qui porte, à une extrémité ii) un groupe glycosyle jouant le rôle de substrat pour l'enzyme cible et, à l'autre, iii) un groupe OR1 qui, quand il est libéré sous sa forme hydroxylée HOR1 par ladite fragmentation, en milieux aqueux, appartient à la classe des fluorophores ESIPT.

Le tandem d'espaceurs comprend un espaceur de type éliminant et un espaceur de type cyclisant et préorganisé pour une cyclisation. Ce choix particulier de tandem d'espaceurs permet d'obtenir deux propriétés fondamentales pour la sonde moléculaire correspondante: (a) elle la rend insensible à la dégradation spontanée et donc à la production d'un signal fluorescent faussement positif, et (b) elle garantit une cinétique de fragmentation rapide lors de la transformation par l'enzyme cible pour une performance adaptée aux applications dans le domaine des sciences de la vie. Le groupe R0 est capable d'être clivé du reste de la molécule par l'action de la glycosidase cible, ce qui conduit à un intermédiaire instable qui s'immole par des réactions d'élimination et de cyclisation/clivage, spontanément et rapidement, pour libérer un précipité fluorescent et ainsi produire un signal fluorescent. Cette architecture moléculaire unique permet une réponse enzymatique particulièrement rapide, et notamment bien plus rapide que celle obtenue par l'emploi de la sonde décrite dans le document WO 2014/020285.

La présente invention concerne donc les composés de formule (I), pour la mise en oeuvre d'une méthode de détection *in vivo,* chez l'homme, d'une glycosidase. Les composés de formule (I) selon l'invention peuvent également être utilisés pour détecter une glycosidase, *in vivo* chez l'animal.

Selon un autre aspect, l'invention concerne un procédé pour détecter *in vitro* ou *ex vivo* la présence d'une glycosidase au moyen du composé (I) selon l'invention. Plus précisément, l'invention concerne un procédé pour détecter *in vitro* ou *ex vivo* la présence d'une glycosidase comprenant les étapes de :
- mise en contact d'un échantillon suspecté de contenir ladite glycosidase avec un composé (I) selon l'invention,
- application de conditions appropriées pour permettre la formation d'un composé fluorescent, notamment sous la forme d'un précipité, par clivage de la liaison covalente entre O et R0, suivi d'un clivage de la liaison -C(O)-OR1, suite à des réactions d'élimination et de cyclisation du tandem d'espaceurs conduisant à la libération de HOR1, et
- analyse quantitative ou qualitative dudit précipité fluorescent.

Le précipité qui peut être obtenu à partir des composés de formule (I) selon l'invention, par clivage de la liaison covalente entre O et R0, suivi d'un clivage de la liaison -C(O)-OR1 suite à une élimination et une cyclisation du tandem d'espaceurs, est fortement fluorescent, alors que le composé de formule (I) correspondant est peu fluorescent ou pas fluorescent du tout. Les composés selon l'invention, qui sont des substrats d'enzyme glycosidase, se comportent comme des sondes fonctionnant selon le mode éteint/allumé.

En particulier, le procédé de détection selon l'invention peut être mis en oeuvre dans des conditions physiologiques, notamment dans un milieu aqueux tamponné à un pH de l'ordre de 7,4.

L'invention concerne encore les composés de formule (II), intermédiaires dans la synthèse des composés de formule (I) : 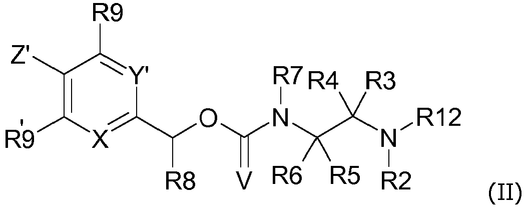 dans laquelle :
- R2, R3, R4, R5, R6, R7, R8, R9, R'9 et V sont tels que définis pour les composés de formule (I),
- R12 représente un atome d'hydrogène, ou un groupe protecteur des fonctions amine,
- X, Y' et Z' sont tels que :
   ∘ soit X représente CR10, Y' représente CR'10 et Z' représente OR'0,
   ∘ soit X représente CR10, Y' représente COR'0 et Z' représente R'10,
   ∘ soit X représente CR10, Y' représente un atome d'azote et Z' représente OR'0,
   ∘ soit X représente un atome d'azote, Y' représente COR'0 et Z représente R10,

   avec R'0 représentant un groupe R0 dont toutes les fonctions alcools sont protégées par un groupe protecteur, et R0, R10 et R'10 étant tels que définis pour les composés de formule (I),
   sous la forme d'un mélange d'isomères optiques selon toutes proportions, ou sous une forme enrichie en un isomère optique.

L'invention concerne encore un procédé de préparation d'un composé (I) comprenant les étapes suivantes :
- disposer d'un composé (II) tel que défini dans le cadre de l'invention,
- disposer d'un composé (III) de formule : 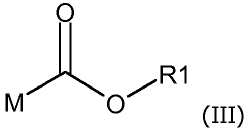 avec R1 tel que défini pour les composés de formule (I) et M représentant un groupe partant, notamment un atome d'halogène, et en particulier un atome de chlore, un groupe imidazolyle ou un paranitrophénoxy, et de préférence M représentant un atome de chlore,
- obtenir le composé (IV) par réaction d'addition dudit composé (II) sur ledit composé (III), ledit composé (IV) ayant pour formule : 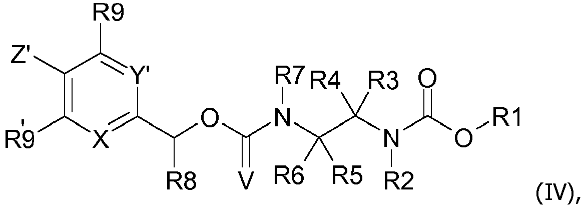 dans lequel R1, R2, R3, R4, R5, R6, R7, R8, R9, R'9, V, X, Y' et Z' sont tels que définis pour les composés de formule (I) et (II), et
- déprotéger les fonctions alcools présentes dans le groupe R'0 dudit composés (IV) pour obtenir ledit composé (I).

Les différents composés selon l'invention peuvent se trouver sous toutes les formes d'isomères optiques possibles, éventuellement en mélange selon toutes proportions, à moins qu'il n'en soit spécifié autrement. Selon un mode de réalisation particulier, les composés selon l'invention comportant un carbone asymétrique se trouvent sous une forme racémique, les formes R et *S* se trouvant en proportions sensiblement égales. Selon un autre mode de réalisation, les composés de formule (I) de l'invention peuvent se trouver sous une forme enrichie en un diastéréoisomère ou énantiomère, avec un excès diastéréoisomérique ou énantiomérique supérieur à 80%, voire même supérieure à 95%, voire sous une forme isomérique pure c'est-à-dire avec un excès diastéréoisomérique ou énantiomérique supérieur à 99 %.

Les composés (I) sont isolés sous une forme enrichie en un diastéréoisomère ou énantiomère par les techniques classiques de séparation : on pourra utiliser, par exemple des recristallisations fractionnées d'un sel du racémique avec un acide ou une base optiquement active dont le principe est bien connu ou, le plus souvent, les techniques classiques de chromatographies sur phase chirale ou non chirale.

Le cas échéant lorsque les composés selon l'invention comprennent une fonction salifiables, ils peuvent se trouver sous la forme d'un sel, notamment d'un chlorhydrate ou d'un trifluoroacétate. L'invention va être décrite de manière plus détaillée. Auparavant, certains termes utilisés vont être définis.

### Définitions

Par « hétérocycle aliphatique », on entend au sens de la présente invention un cycle saturé, substitué ou non substitué, comportant de 3 à 20 chaînons, de préférence de 5 à 10 chaînons, et mieux encore 5, 6, 7 ou 8 chaînons, et comportant au moins un hétéroatome, tel que O, N ou S.

Par « carbocycle aliphatique », on entend au sens de la présente invention un cycle saturé, substitué ou non substitué, comportant de 3 à 20 chaînons, de préférence de 5 à 10 chaînons, et mieux encore 5, 6, 7 ou 8 chaînons, constitué exclusivement par des atomes de carbone. Par « alkyle », on entend une chaîne hydrocarbonée saturée qui peut être linéaire ou ramifiée. De préférence, le terme alkyle désigne, à moins qu'il n'en soit spécifié autrement, un groupe alkyle comprenant de 1 à 12 atomes de carbone et préférentiellement de 1 à 6 atomes de carbone, et notamment un groupe (C1-C4)alkyle. Méthyle, éthyle, n-propyle, isopropyle, iso-butyle et tert-butyle sont des exemples de groupes (C1-C4)alkyle (alkyle avec 1 à 4 atomes de carbone).

Par « alkylène », on entend un groupe alkyle divalent.

Par « aryle », on entend un cycle mono-, bi- ou polycyclique, hydrocarboné insaturé comportant, à moins qu'il n'en soit spécifié autrement, de 5 à 24 chaînons, de 5 à 20 chaînons, de préférence de 5 à 15 chaînons, et comprenant au moins un cycle aromatique alternant liaisons simples et liaisons doubles. A titre d'exemple de groupe aryle, on peut citer les groupes phényle, naphtyle, anthrancényle, phénanthrényle et cinnamyle. Le terme aryle inclut également de tels cycles mono-, bi- ou polycycliques, hydrocarbonés insaturés, dont l'un des carbones constitutifs se trouve sous la forme carboxy -C(O)- tel que le 1H-phénalène-1-one (CAS no. 548-39-0).

Par « arylène », on entend un groupe aryle divalent.

Par « hétéroaryle », on entend un carbocycle mono-, bi- ou polycyclique, comportant, à moins qu'il n'en soit spécifié autrement, de 5 à 24 chaînons, de préférence de 6 à 20 chaînons, préférentiellement de 6 à 15 chaînons et comprenant au moins un groupe aromatique et au moins un hétéroatome, choisi parmi les atomes d'oxygène, azote ou soufre, intégré au sein du carbocycle. A titre d'exemple de groupe hétéroaryle, on peut citer les 2-, 3- ou 4-pyridinyle, 2- ou 3-furoyle, 2- ou 3-thiophényle, pyrrolyle, imidazolyle, pyrazolyle, thiazolyle, benzothiazolyle, oxazolyle, benzoxazolyle, isoxazolyle, pyridinyle, pyrazinyle, pyrimidinyle, tétrazolyle, thiadiazolyle, oxadiazolyle, triazolyle, pyridazinyle, indolyle, oxanyle, 4(1H)-quinolinonyle, dibenzothiophényle, dibenzofuranyle, et 9H-carbazolyle. Le terme hétéroaryle inclut également lesdits groupes dont l'un des carbones constitutifs se trouve sous la forme carboxy -C(O)- tel que le 4(3H)-pyrimidinonyle ou le 4(3H)-quinazolinonyle.

Lorsqu'il est indiqué qu'un groupe est substitué sans plus de précision, cela signifie qu'il est substitué par un ou plusieurs substituants, notamment choisis parmi les atomes de chlore, brome, iode ou fluor, les groupes cyano, alkyle, trifluoroalkyle, trifluorométhyle, alcényle, alcynyle, cycloalkyle, aryle, hétéroaryle, hétérocycloalkyle, amino, alkylamino, dialkylamino, hydroxy, alcoxy, aryloxy, alcoxycarbonyle, aryloxycarbonyle, lesdits groupes pouvant eux-mêmes être substitués. Les termes utilisés pour la définition de ces substituants sont ceux usuellement reconnus par l'homme du métier.

Par « alcoxy » et « aryloxy », on entend respectivement un groupe -O-alkyle et -O-aryle, avec alkyle et aryle tels que définis dans le cadre de la présente invention.

Par « haloalkyle », on entend une chaîne hydrocarbonée, saturée, linéaire ou ramifiée, dans laquelle au moins un atome d'hydrogène a été remplacé par un atome d'halogène.

Par « glycosidase », on entend une enzyme glycoside hydrolase qui a la capacité de catalyser l'hydrolyse de liaisons glycosidiques, de manière à libérer au moins un composé osidique.

Par groupe « glycosyle », on entend tout sucre mono- ou poly-saccharide lié au reste de la molécule par un lien glycosyle, c'est-à-dire via son carbone anomérique. Le carbone anomérique peut adopter la configuration alpha ou beta. A titre d'exemple de groupe glycosyle, on peut citer les groupements mono-glycosyle, c'est-à-dire formé d'une seule unité saccharidique, et polyglycosyle, c'est-à-dire formé de plusieurs unités saccharidiques identiques ou différentes. Les unités saccharidiques peuvent être notamment du type hexoses ou pentoses, et choisies parmi le galactose, le glucose, le mannose, le gulose, l'allose, l'altrose, l'idose, le talose, le fucose, le fructose, l'arabinose, le lyxose, le ribose et le xylose, par exemple. Les unités saccharidiques peuvent être de stéréochimie L ou D.

De manière classique, le terme « alcènyle » désigne une chaîne hydrocarbonée, linéaire ou ramifiée comprenant au moins une double liaison carbone-carbone, et présentant, à moins qu'il n'en soit spécifié autrement, de 2 à 20 atomes de carbone, et de préférence de 2 à 6 atomes de carbone.

Dans le cadre de la présente invention, le terme « alcénylène » désigne un groupe alcényle divalent.

Le terme « alcynyle » désigne une chaîne hydrocarbonée, linéaire ou ramifiée, comprenant au moins une triple liaison carbone-carbone, et présentant, à moins qu'il n'en soit spécifié autrement, de 2 à 12 atomes de carbone, et de préférence de 2 à 6 atomes de carbone.

Par « alcynylène », on entend un groupe alcynyle divalent.

Par « groupe hydrosolubilisant », on entend un groupe hydrophile qui permet d'améliorer la solubilité de la sonde dans un milieu aqueux, par rapport notamment à une sonde qui n'en diffère que par le remplacement du groupe hydrosolubilisant pas un atome d'hydrogène.

La « fluorescence » est la propriété par laquelle une molécule qui est excitée par de la lumière d'une longueur d'onde donnée émet de la lumière à une plus grande longueur d'onde. La fluorescence est un phénomène qui résulte de l'interaction d'un fluorophore avec un photon incident. Ce processus est appelé excitation. L'absorption du photon amène un électron dans le fluorophore à passer de son état fondamental à un niveau d'énergie supérieur. Ensuite, l'électron revient à son niveau original en émettant un photon. Ce processus est appelé émission de fluorescence. Le fluorophore émet ensuite de la lumière à une plus grande longueur d'onde que celle du photon absorbé. Ceci est dû simplement au fait que l'énergie du photon émis est inférieure à celle du photon absorbé, du fait de la dissipation d'énergie pendant la durée de vie de l'état excité. Cette définition est donnée dans la demande de brevet WO 2004/058787.

Les composés (I) selon l'invention sont appelés « substrat de glycosidase » car ils sont transformés en une autre substance pendant une réaction chimique, en particulier une hydrolyse, catalysée par une glycosidase. Pendant une telle réaction en milieu aqueux, les composés (I) (appelés également « sonde ») sont clivés sous l'action de la glycosidase cible, ce qui conduit à la formation d'un précipité fluorescent et d'un produit non fluorescent.

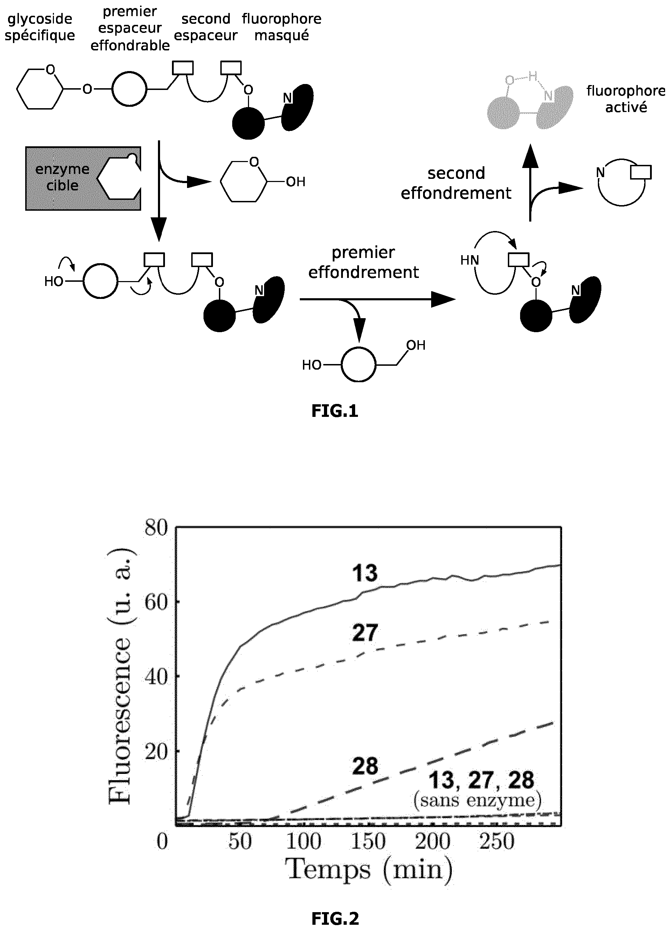

Le « tandem d'espaceurs » au sens de la présente invention est le fragment du composé (I) qui porte à une extrémité un groupe glycosyle -R0 et à l'autre extrémité un groupe -OR1 qui, une fois libéré par hydrolyse, appartient à la classe des fluorophores ESIPT. Ce tandem d'espaceur est constitué d'un premier espaceur de type éliminant (qui va subir une réaction d'élimination suite à l'hydrolyse libérant R0) et un deuxième espaceur de type cyclisant (qui va cycliser suite à l'élimination de l'espaceur de type éliminant, permettant alors de générer HOR1). La **figure 1** représente le mécanisme de dégradation, à partir des composés de formule (I), faisant appel au tandem d'espaceur selon l'invention.

### Composés de formule (I)

La présente invention concerne les composés de formule (I) : 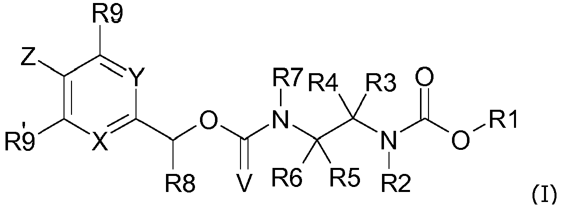 dans laquelle :
- R1 est tel que HOR1, obtenu après clivage de la liaison -C(O)-OR1 présente sur la formule (I), appartient à la classe des fluorophores conduisant à un transfert de proton intramoléculaire dans un état excité, nommés ESIPT, R1 étant un groupe aromatique avec -OR1 qui répond à la formule (A1) telle que définie dans la revendication 1 et telle que détaillée ci-après,
- R2, R3 et R4 sont définis comme suit :
   ∘ soit R2 est un (C1-C4)alkyle, R3 est un (C1-C4)alkyle ou un atome d'hydrogène, et R4 est un (C1-C4)alkyle,
   ∘ soit R3 est un (C1-C4)alkyle ou un atome d'hydrogène et R2 et R4 sont liés entre eux et forment avec les atomes de carbone et d'azote auxquels ils sont liés un hétérocycle aliphatique, cet hétérocycle pouvant être substitué par un groupement hydrosolubilisant,
   ∘ soit R2 est un (C1-C4)alkyle et R3 et R4 sont liés entre eux et forment avec l'atome de carbone auxquels ils sont liés un carbocycle aliphatique,
- R5 et R6 sont identiques ou différents et représentent indépendamment l'un de l'autre un atome d'hydrogène, un (C1-C4)alkyle, ou un (C5-C10)aryle,
- R7 est un atome d'hydrogène, ou un groupe choisi parmi les (C1-C4)alkyle et (C1-C4)alcoxy,
- R8 représente un atome d'hydrogène, ou un groupe (C1-C10)alkyle, substitué ou non substitué, ou un groupe -D1-D2-D3 avec :
   ∘ D1 représentant un groupe triazolyle ou -CH₂-triazolyle,
   ∘ D2 représentant un groupe (C1-C10)alkylène, (C1-C10)alcènylène, ou (C1-C10)alcynylène, lesdits groupes étant éventuellement interrompus par un ou plusieurs hétéroatomes choisis parmi O ou N, un groupe glycosyle divalent, un groupe -O-(CHR-CHR'-O)ₙ- ou -N-(CHR-CHR'-O)ₙ- avec n étant un entier naturel variant de 1 à 20, R et R', identiques ou différents, représentant H ou CH₃ à condition que R et R' ne soient pas simultanément CH₃, un acide aminé ou un peptide, ou une combinaison de ces groupes,
   ∘ D3 représentant un motif maléimidocaproyl, acide aminé, peptide, acide folique, anticorps ou fragment d'anticorps lié à D2, par une fonction acide carboxylique qu'il comprend, formant un lien ester ou amide,
- R9 et R'9, identiques ou différents, représentent un atome d'hydrogène, ou un groupe électro-attracteur tel qu'un atome d'halogène, ou un groupe choisi parmi - NO₂, -CN ou -NH-C(O)-CH₂-Ab avec Ab représentant un anticorps,
- V représente un atome d'oxygène ou un atome de soufre,
- X, Y et Z sont tels que :
   ∘ soit X représente CR10, Y représente CR'10 et Z représente OR0,
   ∘ soit X représente CR10, Y représente CORO et Z représente R'10,
   ∘ soit X représente CR10, Y représente un atome d'azote et Z représente OR0,
   ∘ soit X représente un atome d'azote, Y représente CORO et Z représente R10
   avec :
   ∘ R0 représentant un groupement glycosyle lié par son carbone anomérique au reste de la molécule de formule (I), et
   ∘ R10 et R'10, identiques ou différents, représentant un atome d'hydrogène ou un groupe électro-donneur tel qu'un (C1-C20)alkyle, un (C5-C24)aryle, ou un (C1-C20)alcoxy,
   sous la forme d'un mélange d'isomères optiques selon toutes proportions, ou sous une forme enrichie en un isomère optique.

Le groupe OR1 est sélectionné de manière à ce que le précipité fluorescent obtenu qui correspond à R1OH, libéré après clivage de la liaison -C(O)-OR1, soit un fluorophore ESIPT, avec le groupement -OR1 qui répond à la formule (A1): 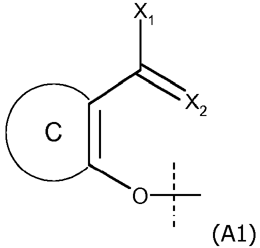 dans laquelle :
- soit X2 est un atome d'oxygène et X1 est un groupe -NH₂, -OH, -SH, (C1-C20)alkyle, (C5-C24)aryle, -O-(C1-C20)alkyle, -O-phényle, -NH-(C1-C20)alkyle ou -NH-phényle, -S-(C1-C20)alkyle ou -S-(C5-C24)aryle, lesdits groupes alkyle et phényle pouvant être substitués ou non substitués,
   soit X2 représente un atome d'azote et est lié à X1 qui représente alors CH, O, S, N ou NH pour former un (C5-C24)hétéroaryle substitué ou non substitué, 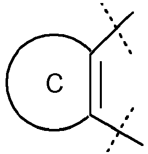
- représente un (C5-C24)aryle ou un (C5-C24)hétéroaryle, substitué ou non substitué, par exemple choisi parmi les groupes phényle, naphtyle, et : 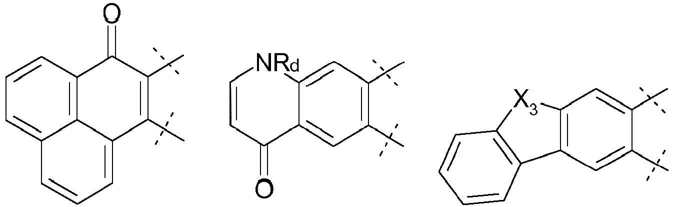 lesdits groupes pouvant être substitués ou non substitués,
   avec X3 qui représente S, O ou NRd et Rd qui représente un atome d'hydrogène ou un groupe (C1-C4)alkyle.

Les fluorophores ESIPT montrent un déplacement de Stokes qui dépassent les 100 nm et accèdent souvent 200 nm. Tous les fluorophores ESIPT perdent cette émission de fluorescence correspondant à un déplacement de Stokes supérieur à 100 nm, si leur groupement OH du type phénolique, donnant lieu au transfert de proton intra-moléculaire à l'état excité, est alkylé, acylé ou autrement fonctionnalisé. Cette fonctionnalisation empêche de transférer un atome d'hydrogène à l'hétéroatome X2 sur l'illustration donnée avec la formule (A1), lors de l'excitation par irradiation, et ainsi empêche l'émission de fluorescence caractéristique du processus de transfert de proton. L'incorporation de l'hydroxyle HOR1 dans le groupement carbamate du composé de formule (I) empêche le transfert de proton. Le transfert de proton intramoléculaire pourra ensuite se produire grâce au groupement hydroxy obtenu suite à la coupure de la liaison -C(O) - OR1. Le plus souvent, le groupe R1 correspond à un groupe phényle qui est non substitué ou substitué et/ou qui est fusionné avec un ou plusieurs carbocycles insaturés, comprenant éventuellement un hétéroatome tel que l'azote. Ce dérivé phénoxy OR1, lorsqu'il n'est pas lié au substrat, correspond sous sa forme protonée à un dérivé phénolique HO-R1 qui appartient à la classe des fluorophores ESIPT.

Des dérivés -OR1 correspondent, par exemple, à l'une des structures préférées (A2) ou (A3) suivantes : 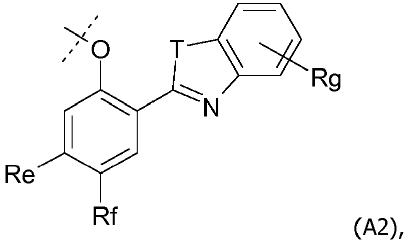 dans laquelle :
∘ T est -NH-C(O)-, -S-, -O-, -NH-, -N((C1-C20)alkyle)- ou -N((C5-C24)aryle)-,
∘ Re est un atome d'hydrogène ou un substituant carboné attracteur d'électrons comme -CN ou -COORh, avec Rh qui représente un groupe (C1-C4)alkyle, ou bien Re est -CONRiRj, avec Ri et Rj, identiques ou différents, qui représentent un atome d'hydrogène ou un groupe (C1-C4)alkyle, ou bien Re est -CF₃, ou un groupe 2-oxazolyle, 2-thiazolyle, 2-imidazolyle, 2-benzoimidazolyle, 4-pyrimidinon-2-yle ou quinazolinon-2-yle,
∘ Rf est un atome d'hydrogène, un atome de chlore, brome, iode ou fluor, -OH, -NH₂, -NRkRl, -NHRk ou -ORk, avec Rk et Rl identiques ou différents qui représentent chacun indépendamment un groupe (C1-C4)alkyle,
∘ ou bien Re et Rf sont liés entre eux pour former une chaîne hydrocarbonée comprenant 4 ou 5 chaînons, saturée ou insaturée, substituée ou non substituée, éventuellement interrompue par un ou plusieurs hétéroatomes choisis parmi N, S et O,
∘ Rg est un atome d'hydrogène, Br, Cl, I ou F, 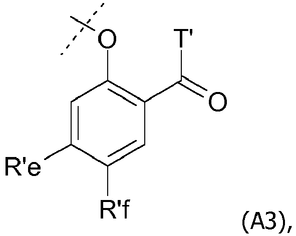 dans laquelle :
∘ T' est -NH₂, -OH, un groupe (C5-C24)aryle, un groupe (C1-C4)alkyle, -SH, -NHR'g, -OR'g, -NR'gRh'ou -SR'g, R'g et Rh', identiques ou différents, représentant un groupe (C1-C4)alkyle ou aryle,
∘ R'e est atome d'hydrogène ou un substituant carboné attracteur d'électrons comme -CN, ou -COOR'i, avec R'i qui représente un groupe (C1-C4)alkyle, ou R'e est -CONR'jR'k, avec R'j et R'k, identiques ou différents, qui représentent un atome d'hydrogène ou un groupe (C1-C4)alkyle, ou bien R'e est -CF₃, ou un groupe 2-oxazolyle, 2-thiazolyle, 2-imidazolyle, 2-benzoimidazolyle, 4-pyrimidinon-2-yle, ou quinazolinon-2-yle,
∘ R'f est un atome d'hydrogène, de chlore, de brome, d'iode ou de fluor, -OH, -NH₂, -NR'IR'm ou -OR'l, avec R'l et R'm, identiques ou différents, qui représentent un groupe (C1-C4)alkyle,
∘ ou bien R'e et R'f sont liés entre eux pour former une chaîne hydrocarbonée comprenant 4 ou 5 chaînons, saturée ou insaturée, substituée ou non substituée, éventuellement interrompue par un ou plusieurs hétéroatomes choisis parmi N, S et O.

On pourra notamment se référer aux demandes WO 2013/045854, WO 2014/020285 et WO 2015/197981 qui donnent des exemples de tels fluorophores ESIPT qui peuvent être utilisés dans la présente invention.

Selon un mode de réalisation particulier de l'invention, R1 est un groupe aromatique avec -OR1 qui répond à à la formule (A5) suivante :

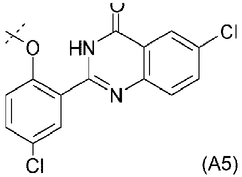

Le très grand déplacement de Stokes de tels fluorophores (approximativement 170 nm pour A5) ou de tout analogue de la HPQ contribuera à l'excellente sensibilité de la sonde et rendra le fluorophore libéré aisément distinguable de la fluorescence native pouvant provenir de l'échantillon biologique sur laquelle l'analyse va être menée.

Selon un mode de réalisation de l'invention, R2 est un (C1-C4)alkyle, R3 est un (C1-C4)alkyle ou un atome d'hydrogène, et R4 est un (C1-C4)alkyle. Selon un mode particulier de réalisation, R2, R3 et R4, identiques ou différents, représentent un groupe (C1-C4)alkyle, par exemple méthyle ou éthyle. Selon un mode particulier de réalisation, R2 = R3 = R4 = -CH₃.

Selon un autre mode de réalisation, R3 est un (C1-C4)alkyle ou un atome d'hydrogène et R2 et R4 sont liés entre eux et forment avec les atomes de carbone et d'azote auxquels ils sont liés un hétérocycle aliphatique, cet hétérocycle pouvant être substitué par un groupement hydrosolubilisant. Selon un mode particulier de réalisation, R3 est un atome d'hydrogène ou un groupe (C1-C4)alkyle, de préférence un atome d'hydrogène, et R2 et R4 sont liés entre eux et forment un enchaînement -(CH₂)ₘ- avec m = 3, 4 ou 5. Selon un autre mode particulier de réalisation, R3 est un atome d'hydrogène ou un groupe (C1-C4)alkyle, de préférence un atome d'hydrogène, et R2 et R4 sont liés entre eux et forment un enchaînement -CH₂CH₂-NR11-CH₂- dans le sens de R2 vers R4, R11 représentant un atome d'hydrogène ou -(L)n-GP avec n qui est égal à 0 ou 1, L un bras de liaison et GP un groupe hydrosolubilisant, i.e. le composé (I) a alors pour formule :

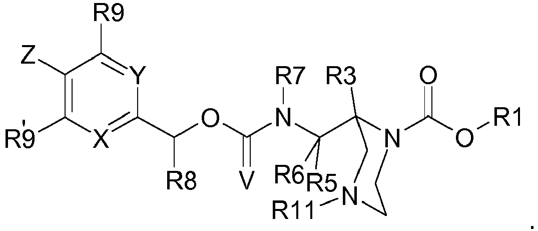

Bien souvent, pour des raisons de synthèse, n = 1 et L est un bras de liaison, et notamment un bras -(L1)ₘ₁-(L2)ₘ₂-(L'1)_{m'1}- (dans le sens pipérazine -> groupe GP) avec :
- L1 et L'1, identiques ou différents, qui sont choisis parmi -O-, -NH-, -N(C1-C6)alkyl)-, -N(phényl)-, -N(aryl)-, -C(O)-, -C(O)O-, -OC(O)-, -OC(O)-O-, -NHC(O)-O-, -OC(O)-NH-, -NHC(O)-NH-, -S-, -SO₂-, -N=N-, -NHC(O)- et -CONH- ;
- L2 qui est choisi parmi les groupes divalents suivants : (C1-C20)alkylène, (C1-C20)alcénylène, (C1-C20)alcynylène, (C6-C24)arylène, (C7-C44)alkylarylène, (C7-C44)alcénylarylène, (C7-C44)alcynylarylène, (C7-C44)alkylcycloalkylène, (C7-C44)alcénylcycloalkylène, (C7-C44)alcynylcycloalkylène, (C7-C44) alkylhétérocycloalkylène, (C7-C44)alcénylhétérocycloalkylène, (C7-C44)alcynylhétérocycloalkylène ; lesdits groupes pouvant être interrompus ou se terminer par un groupe triazole, et pouvant être non substitués ou substitués, notamment par un ou plusieurs substituants choisis parmi les (C1-C10)alcoxy, (C1-C10)alkyle, (C6-C10)aryle, amido, imido, phosphido, nitrido, (C1-C10) alcényle, (C1-C10) alcynyle et -OH ; et
- m1, m'1 et m2, identiques ou différents, qui sont égaux à 0 ou 1.

Le bras L, lorsqu'il est présent, sera choisi pour éloigner le groupe GP de la pipérazine ou pour des questions de synthèse. Selon un mode de réalisation préféré, L représente -(L1)ₘ₁-(L2)ₘ₂-(L'1)_{m'1} avec L1 = -C(O)-, m1 = m2 = 1, m'1 = 1 ou 0 et L2 et L'1 tels que définis précédemment, et notamment L représente -C(O)-(CH₂)ₚ-L3- avec p qui est égal à 1, 2, 3 ou 4 et L3 qui est un groupe triazole et notamment un groupe 1*H*-1,2,3-triazole.

GP est un groupe hydrosolubilisant. A titre d'exemple de groupe hydrosolubilisant, on peut citer les groupes capables de former une espèce chargée en solution aqueuse. A titre d'exemple de groupe hydrosolubilisant GP, on peut citer les fonctions F1 choisies parmi les amine (primaire, secondaire, ou tertiaire), amidine, guanidine ou tétrazole ; les fonctions F2 cationiques ou anioniques, et notamment les groupes type ammonium, carboxylate, sulfonate ou phosphate ; les groupements comprenant une ou plusieurs de ces fonctions F1 et/ou F2 ; les polyéthylèneglycols ; les sucres ou polysaccharides tels que le glucose, le galactose et le mannose ; les groupes peptidiques tels que la poly-lysine, la poly-arginine, les TAT-peptides. A titre d'exemple de fonctions amine, on peut citer -NH₂, -NH(C1-C4)alkyle, et les dialkylamines dans lesquelles les groupes alkyle sont identiques ou différents et comprennent de 1 à 4 atomes de carbone.

Selon un autre mode de réalisation, R2 est un (C1-C4)alkyle et R3 et R4 sont liés entre eux et forment avec l'atome de carbone auxquels ils sont liés un carbocycle aliphatique, de préférence un cyclohexyle.

Ces deux manières de préorganiser l'espaceur pour la cyclisation, consistant soit à introduire deux substituants alkyle (ou formant un carbocycle) sur le carbone en alpha du groupement - N-C(V)-O-, soit à inclure la liaison entre l'azote du groupement -N-C(V)-O- et son carbone alpha dans un hétérocycle, accélèrent le processus d'immolation.

Selon un mode de réalisation, R5 et R6 sont identiques et représentent un atome d'hydrogène. Selon un mode de réalisation, R7 représente un atome d'hydrogène ou un groupe (C1-C4)alkyle tel qu'un méthyle, et de préférence un atome d'hydrogène.

Selon un mode de réalisation, R5, R6 et R7 représentent chacun un atome d'hydrogène.

Selon un mode de réalisation, R8 un atome d'hydrogène.

Selon un autre mode de réalisation, R8 est un groupe -D1-D2-D3, avec D1, D2 et D3 tels que définis dans le cadre de la présente invention. Selon ce mode de réalisation, D3 peut notamment représenter un motif acide folique, anticorps ou peptide, qui sont des groupements ciblant un récepteur cellulaire, afin d'améliorer la sélectivité des composés de formule (I) pour certaines cellules particulières.

Selon un mode de réalisation particulier de l'invention, R8 est un groupe -D1-D2-D3, avec D1 représentant un groupe -CH₂-triazolyle, D2 représentant un groupe (C1-C10) alkylène, de préférence interrompu par un ou plusieurs hétéroatomes choisis parmi O ou N, de préférence O, et D3 réprésentant un motif maléimidocaproyl, acide aminé, peptide, acide folique, anticorps ou fragment d'anticorps, de préférence acide folique, lié à D2 par une fonction acide carboxylique qu'il comprend de manière à former un lien ester ou amide, de préférence une fonction amide. Selon un mode particulier de réalisation, R8 est un groupe -D1-D2-D3 de formule suivante :

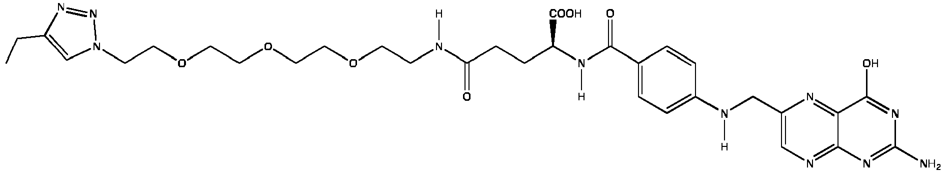

Selon un mode de réalisation, au moins un des groupes R9 ou R'9 représente un atome d'halogène, un groupe -NO₂, ou un groupe -CN, de préférence un groupe -NO₂. Selon un mode de réalisation préféré, R9 représente un atome d'hydrogène et R'9 représente un atome d'halogène, un groupe -NO₂ ou un groupe -CN, de préférence un groupe -NO₂.

Selon un mode de réalisation, R10, et le cas échéant R10', représente(nt) un atome d'hydrogène. Selon un mode de réalisation, V représente un atome d'oxygène.

Les groupements R0 ont la caractéristique de pouvoir être clivés du reste de la molécule sous l'action d'une enzyme glycosidase. L'enzyme joue le rôle de catalyseur de la coupure entre R0 et l'atome d'oxygène auquel il est lié. Une telle coupure peut être la conséquence d'une hydrolyse en milieu aqueux pour laquelle l'enzyme glycosidase va jouer le rôle de catalyseur. C'est pourquoi ladite enzyme glycosidase est dite catalytiquement active.

R0 représente un groupement glycosyle lié par son carbone anomérique au reste de la molécule. R0 est apte à être clivé du reste de la composé de formule (I) sous l'action catalytique d'une enzyme glycosidase, en particulier en milieu aqueux. A titre d'exemples d'enzyme glycosidase qui peuvent êtres ciblés par les sondes fluorogènes selon l'invention, on peut citer la N-acétyl-β-galactosaminidase ; la N-acétyl-β-glucosaminidase ; la·α-amylase ; la·α-arabinofuranosidase ; la·α-arabinosidase ; la·β-cellobiosidase ; la·β-chitobiosidase ; la·α-galactosidase ; la·β-galactosidase ; la·α-glucosidase ; la·β-glucosidase ; la·β-glucuronidase ; la·α-maltosidase ; la·α-mannosidase ; la·β-mannosidase ; la·β-xylosidase ; la·β-D-fucosidase ; la·α-L-fucosidase ; la·β-L-fucosidase ; la L-iduronidase ou de la cellulase (Orenga, S., James, A. L., Manafi, M., Perry, J. D., & Pincus, D. H. (2009). Enzymatic substrates in microbiology. Journal of Microbiological Methods, 79(2), 139-155).

Le groupement R0 sera, de préférence, choisi de manière à être spécifique d'une glycosidase d'intérêt. En revanche, certaines glycosidases ont la capacité de cliver un ensemble de groupements R0 différents ; parmi elles, on peut citer l'hexosaminidase.

Tous les groupements glycosyle possibles qui correspondent à un groupe R0-O clivable en milieu aqueux en présence d'une glycosidase peuvent être utilisés en tant que R0. Les unités glycosyle peuvent être fonctionnalisées ou non, notamment avec un groupement acétyl ou amino. Les N-acétylhexosamines sont des exemples de groupe glycosyle. Le plus souvent, le groupe glycosyle comprendra de 1 à 50 unités saccharidiques. Dans le cas d'un polyglycosyle, il pourra s'agir d'un homopolymère ou d'un copolymère avec une structure aléatoire, alternée ou block.

Des exemples de tels groupes R0 sont donnés ci-après : les groupements mono-glycosylés choisis parmi le galactosyle, le glucosyle, le mannosyle, le gulosyle, l'allosyle, l'altrosyle, l'idosyle, le talosyle, le fucosyle, le fructosyle, l'arabinosyle, le lyxosyle, le ribosyle, le xylosyle, le glucuronyle et le N-acétyl-hexosaminyle et les groupements polyglycosylés constitués de plusieurs de ces groupements monoglycosylés identique ou différents.

Selon un mode de réalisation, R0 est un groupe clivable sous l'action d'une glycosidase, choisie parmi la N-acétyl-β-galactosaminidase ; la N-acétyl-β-glucosaminidase ; la α-amylase ; la α-arabinofuranosidase ; la α-arabinosidase ; la β-cellobiosidase ; la β-chitobiosidase ; la α-galactosidase ; la β-galactosidase ; la α-glucosidase ; la β-glucosidase ; la β-glucuronidase ; la α-maltosidase ; la α-mannosidase ; la β-mannosidase ; la β-xylosidase ; la β-D-fucosidase ; la α-L-fucosidase ; la β-L-fucosidase ; la L-iduronidase ou de la cellulase ; et R0 est un groupement mono-glycosylé lié par son carbone anomérique choisi parmi le galactosyle, le glucosyle, le mannosyle, le gulosyle, l'allosyle, l'altrosyle, l'idosyle, le talosyle, le fucosyle, le fructosyle, l'arabinosyle, le lyxosyle, le ribosyle, le xylosyle, le glucuronyle et le N-acétyl-hexosaminyle ou un groupement polyglycosylé constitué de plusieurs, par exemple de 2 à 20, de préférence de 2 à 10, et plus particulièrement de 2 à 6, de ces groupements monoglycosylés identiques ou différents.

Selon un mode de réalisation, R0 est un groupe clivable sous l'action d'une galactosidase, par exemple une β-galactosidase, d'une induronidase, d'une glucosidase, d'une N-acétyl-D-glucosaminidase, d'une N-acétyl-D-galactosaminidase, d'une mannosidase, d'une fucosidase, d'une glucuronidase, notamment d'une β-glucuronidase ou d'une cellulase ; et R0 est un groupement mono-glycosylé, lié par son carbone anomérique, choisi parmi le D-glucuronyle, le L-iduronyle, le D-glucopyranosyle, le D-galactopyranosyle, le N-acétyl-D-glucosaminyle, le N-acétyl-D-galactosaminyle, le D-mannopyranosyle, le L-fucopyranosyle ou un groupement polyglycosylé constitué de plusieurs, par exemple de 2 à 20, de préférence de 2 à 10, et plus particulièrement de 2 à 6, de ces groupements monoglycosylés identiques ou différents.

Selon un mode de réalisation, X, Y et Z sont tels que :
- soit X représente CR10, Y représente CR'10 et Z représente OR0,
- soit X représente CR10, Y représente CORO et Z représente R'10
avec R10, R'10 et R0 tels que définis dans le cadre de l'invention.

Dans le cadre de la présente invention, on utilisera, en particulier, les définitions spécifiques des substituants données en combinaison.

Selon un premier mode de réalisation particulier, le composé (I) est tel que représenté par la formule (Ia) ci-dessous, sous forme d'un mélange d'isomères optiques selon toutes proportions, ou sous une forme enrichie en un isomère optique, où R1, R2, R3, R4, R5, R6, R7, R8, R0 et V sont tels que définis dans le cadre de la présente invention :

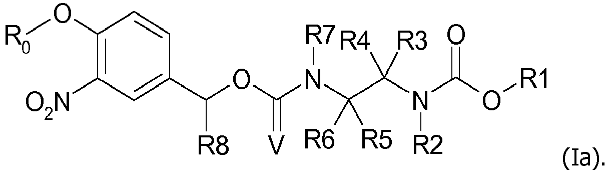

Selon un deuxième mode de réalisation particulier de l'invention, le composé (I) est tel que représenté par la formule (Ib) ci-dessous, sous forme d'un mélange d'isomères optiques selon toutes proportions, ou sous forme enrichie en un isomère optique, où R1, R2, R3, R4, R9, R'9, X, Y et Z sont tels que définis dans le cadre de la présente invention :

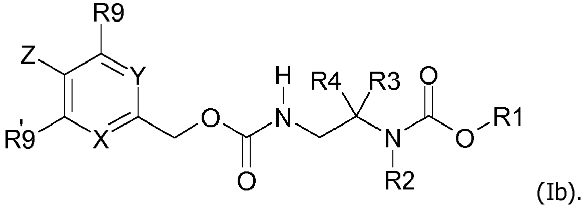

Selon un troisième mode de réalisation, le composé (I) est tel que représenté par la formule (Ic) ci-dessous, sous forme d'un mélange d'isomères optiques selon toutes proportions, ou sous forme enrichie en un isomère optique, où R0 et R1 sont tels que définis dans le cadre de la présente invention :

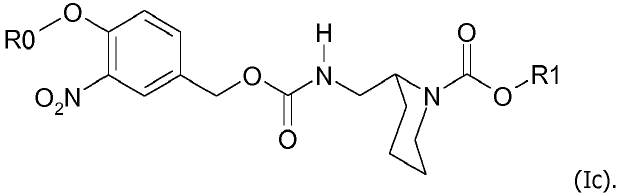

Selon un quatrième mode de réalisation, le composé (I) est tel que représenté par la formule (Id) ci-dessous, sous forme d'un mélange d'isomères optiques selon toutes proportions, ou sous forme enrichie en un isomère optique, où R1, R2, R3, R4, R9, R'9, D1, D2, D3, X, Y et Z sont tels que définis dans le cadre de l'invention :

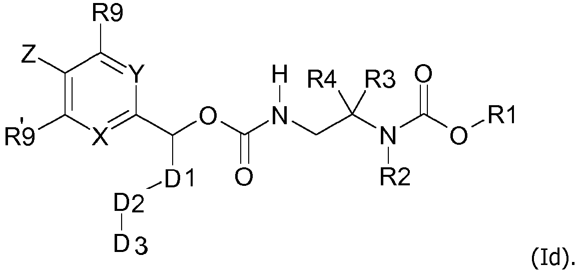

Selon un cinquième mode de réalisation, le composé (I) est tel que représenté par la formule (Ie) ci-dessous, sous forme d'un mélange d'isomères optiques selon toutes proportions, ou sous forme enrichie en un isomère optique, où R0, R1, D1, D2, et D3 sont tels que définis dans le cadre de l'invention :

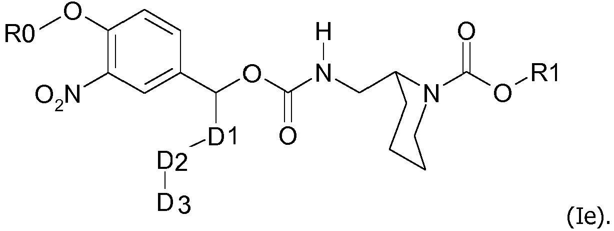

Selon un mode particulier de réalisation, les composés de formule (I) sont tels que représentés par la formule (If) ci-dessous, sous forme d'un mélange d'isomères optiques selon toutes proportions, ou sous forme enrichie en un isomère optique, où R0 est tel que défini dans le cadre de l'invention :

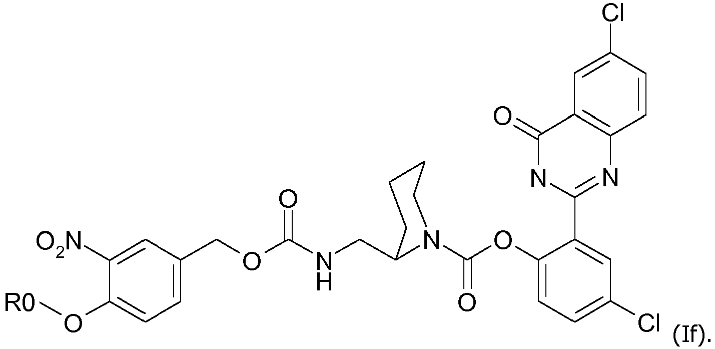

Selon un mode particulier de réalisation, l'invention concerne les composés, sous forme d'un mélange d'isomères optiques selon toutes proportions, ou sous forme enrichie en un isomère optique, choisis parmi : 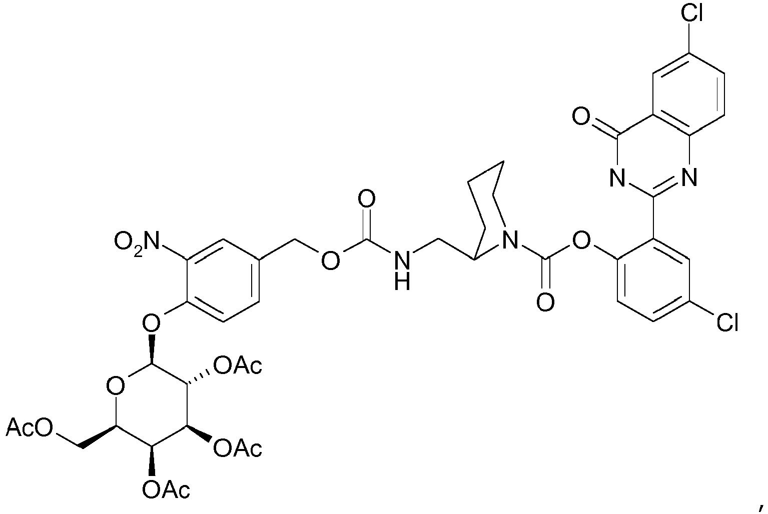 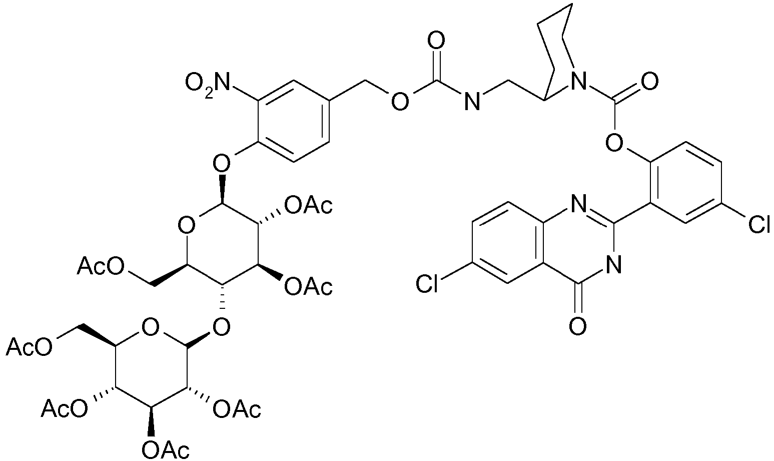 et

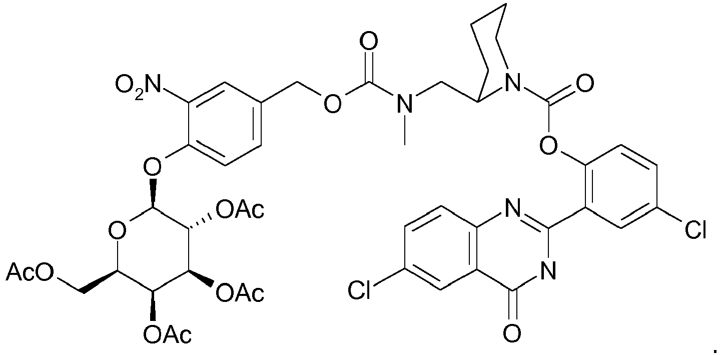

### Composés de formule (II)

La présente invention concerne encore les composés de formule (II) : 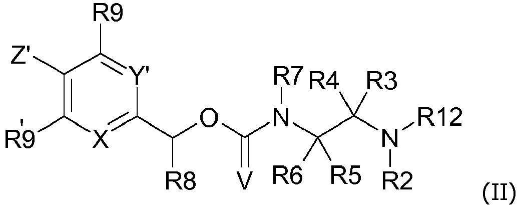 dans laquelle :
- R2, R3, R4, R5, R6, R7, R8, R9, R'9 et V sont tels que définis pour les composés de formule (I),
- R12 représente un atome d'hydrogène, ou un groupe protecteur des fonctions amine,
- X, Y' et Z' sont tels que :
   ∘ soit X représente CR10, Y' représente CR'10 et Z' représente OR'0,
   ∘ soit X représente CR10, Y' représente COR'0 et Z' représente R'10,
   ∘ soit X représente CR10, Y' représente un atome d'azote et Z' représente OR'0,
   ∘ soit X représente un atome d'azote, Y' représente COR'0 et Z représente R10,
   avec R0' représentant un groupe R0 dont toutes les fonctions alcools sont protégées par un groupe protecteur, et R0, R10 et R'10 étant tels que définis pour les composés de formule (I),
sous la forme d'un mélange d'isomères optiques selon toutes proportions, ou sous une forme enrichie en un isomère optique.

Les composés de formule (II) sont des intermédiaires de synthèse des composés de formule (I). Par groupe protecteur des fonctions amine, on entend les groupes protecteurs tels que ceux décrits dans Protective Groups in Organic Synthesis, Greene T.W. et Wuts P.G.M., ed. John Wiley et Sons, 2006 et dans Protective Groups, Kocienski P.J., 1994, Georg Thieme Verlag.

Selon un mode de réalisation, R12 représente un groupe protecteur des fonctions amine. A titre d'exemple, R12 représente un groupe protecteur des fonctions amine choisi parmi les groupes allyle ou carbamate, tel qu'un groupe tert-butoxycarbonyle (Boc), - fluorophényleméthoxycarbonyle (Fmoc), allyloxycarbonyle (Alloc) ou 2,2,2-trichloroéthoxycarbonyle (Troc).

Selon un autre mode de réalisation particulier, R12 représente un atome d'hydrogène.

Selon un mode de réalisation, R'0 représente un groupe R0 dont toutes les fonctions alcools sont protégées par un groupe protecteur des fonctions alcool, de préférence dans les conditions réactionnelles utilisées lors de la réaction entre les composés (II) et (III), et notamment par des groupes silyle tel que les groupes triméthylsilyle, tert-butyldiméthylsilyle, tert-butyldiphénylsilyle et triiso-propylsilyle ; sous forme d'acétal, et notamment de 1,3-dioxolane ; ou sous forme d'ester d'acides gras.

Selon un mode de réalisation de l'invention, le composé (II) est tel que représenté par la formule (IIa) ci-dessous, sous forme d'un mélange d'isomères optiques selon toutes proportions, ou sous une forme enrichie en un isomère optique, où R2, R3, R4, R5, R6, R7, R8, R12, R0 et V sont tels que définis dans le cadre de l'invention :

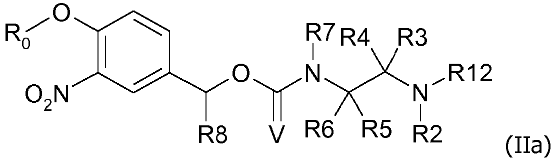

Selon un deuxième mode de réalisation particulier de l'invention, le composé (II) est tel que représenté par la formule (IIb) ci-dessous, sous forme d'un mélange d'isomères optiques selon toutes proportions, ou sous forme enrichie en un isomère optique, où R2, R3, R4, R9, R'9, R12, X, Y et Z sont tels que définis dans le cadre de l'invention :

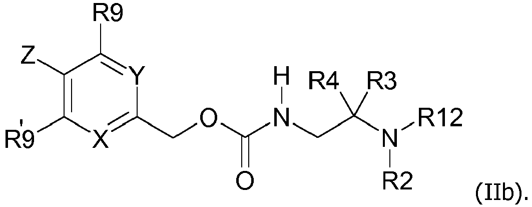

Selon un troisième mode de réalisation, le composé (II) est tel que représenté par la formule (IIc) ci-dessous, sous forme d'un mélange d'isomères optiques selon toutes proportions, ou sous forme enrichie en un isomère optique, où R0 et R12 sont tels que définis dans le cadre de l'invention :

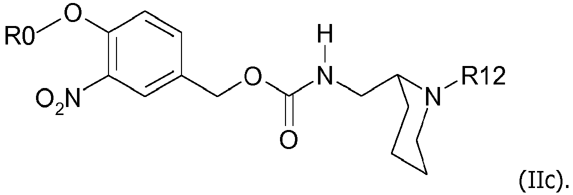

Selon un quatrième mode de réalisation particulier de l'invention, le composé (II) est tel que représenté par la formule (IId) ci-dessous, sous forme d'un mélange d'isomères optiques selon toutes proportions, ou sous forme enrichie en un isomère optique, où R2, R3, R4, R9, R'9, R12, D1, D2, D3, X, Y et Z sont tels que définis dans le cadre de l'invention :

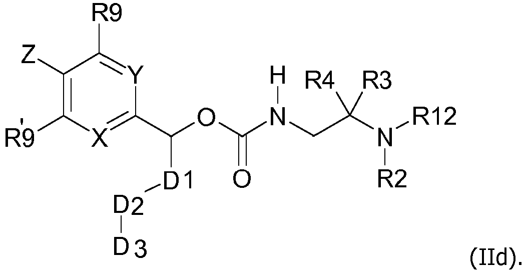

Selon un cinquième mode de réalisation, le composé (II) est tel que représenté par la formule (IIe) ci-dessous, sous forme d'un mélange d'isomères optiques selon toutes proportions, ou sous forme enrichie en un isomère optique, où R0, R12, D1, D2 et D3 sont tels que définis dans le cadre de l'invention :

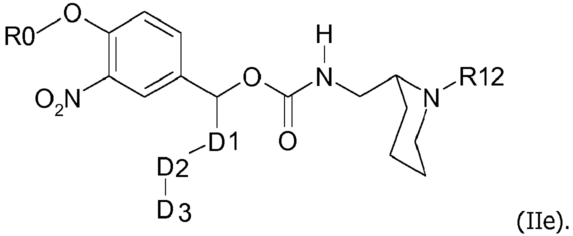

### Procédé de préparation des composés de formule (I)

La présente invention concerne encore un procédé de préparation d'un composé (I), tel que décrit dans le cadre de la présente invention, comprenant les étapes suivantes :
- disposer d'un composé (II) tel que décrit dans le cadre de l'invention,
- disposer d'un composé (III) de formule : 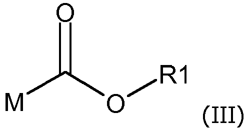 avec R1 tel que défini pour les composés de formule (I), et M représentant un groupe partant, notamment un atome d'halogène, et en particulier de chlore, un groupe imidazolyle ou un paranitrophénoxy, et de préférence M représentant un atome de chlore,
- obtenir le composé (IV) par réaction d'addition dudit composé (II) sur ledit composé (III), ledit composé (IV) ayant pour formule : 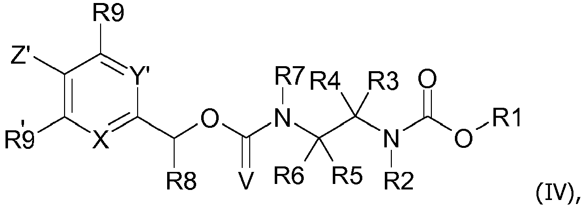 dans laquelle R1, R2, R3, R4, R5, R6, R7, R8, R9, R'9, V, X, Y', et Z' sont tels que définis pour les composés de formule (I) et (II), et
- déprotéger les fonctions alcools présentes dans le groupe R'0 dudit composés (IV) pour obtenir ledit composé (I).

Selon un mode de réalisation, la réaction d'addition du composé (II) sur le composé (III) est réalisée avec un composé (II) dans lequel R12 est un atome d'hydrogène.

Selon un autre mode de réalisation, on dispose d'un composé (II) dans lequel R12 n'est pas un atome d'hydrogène, et une étape de déprotection de la fonction amine du composé (II) est réalisée préalablement à la réaction d'addition du composé (II) sur le composé (III), de sorte à obtenir un composé (II) tel que R12 = H.

Lorsque V = O, le composé (II) peut être avantageusement obtenu selon les étapes suivantes :
- disposer d'un composé (V) de formule suivante : 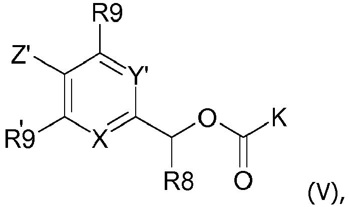
- disposer d'un composé (VI) de formule suivante : 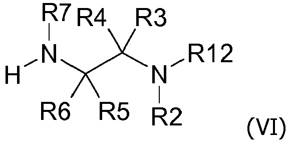
- et obtenir le composé (II) par réaction d'addition dudit composé (VI) sur le composé (V),
dans lesquelles R2, R3, R4, R5, R6, R7, R8, R9, R'9, R12 X, Y' et Z' sont tels que définis dans le cadre de l'invention, et K représente un groupe partant, en particulier un halogène, et notamment le chlore, ou un groupe imidazolyle ou paranitrophénoxy.

Lorsque R8 = D1-D2-D3, R8 peut être introduit par click chemistry. La formation d'un groupe triazole pourra être réalisée par réaction entre des fonctions -N₃ et alcynyle, selon des techniques bien connues de l'homme du métier.

Plus particulièrement, lorsque R8 = D1-D2-D3, avec D1 représentant un groupe -CH₂-triazolyle, D2 représentant un groupe (C1-C10) alkylène, de préférence interrompu par un ou plusieurs hétéroatomes choisis parmi O ou N, de préférence O, et D3 réprésentant un motif acide folique, lié à D2 par une fonction acide carboxylique qu'il comprend de manière à former un lien amide, R8 peut être introduit par réaction entre un alcyne, porté par le précurseur du composé de formule (I), et un azoture selon une réaction de cycloaddition de Huisgens. L'azoture est formé préalablement par réaction entre la fonction acide de l'acide folique et la fonction amine d'un composé de formule H₂N-D2-N₃.

Il est également possible, de manière préférée, d'utiliser les procédés illustrés dans les exemples.

### Détection de la présence de glycosidase

Les composés de formule (I) selon l'invention peuvent être utilisés pour détecter une glycosidase, *in vivo* chez l'animal ou chez l'être humain.

L'administration du composé de formule (I) peut être réalisée par une injection intraveineuse ou intrapéritonéale, ou bien de façon cutanée, en pulvérisant un spray contenant la molécule en solution par exemple.

L'analyse de la fluorescence du composé de formule (I) peut avoir lieu au moyen d'une chambre d'imagerie selon des techniques de type tomographie par fluorescence ou épifluorescence.

L'invention concerne également un procédé pour détecter *in vitro* ou *ex vivo* la présence d'une glycosidase au moyen d'un composé (I) selon l'invention. Plus précisément, l'invention concerne un procédé pour détecter *in vitro* ou *ex vivo* la présence d'une glycosidase comprenant les étapes de :
- mise en contact d'un échantillon suspecté de contenir ladite glycosidase avec un composé (I) selon l'invention,
- application de conditions appropriées pour permettre la formation d'un composé fluorescent, notamment sous la forme d'un précipité, par clivage de la liaison covalente entre O et R0, suivi d'un clivage de la liaison -C(O)-OR1, conduisant à la libération de HOR1 et
- analyse quantitative ou qualitative dudit précipité fluorescent.

L'échantillon peut être tout échantillon biologique approprié, issu d'un homme, d'un animal, d'un végétal ou d'un microorganisme. Dans le cas d'un échantillon issu d'un homme ou d'un animal, il peut s'agir notamment d'un prélèvement de fluide biologique, notamment un échantillon de sang total, de sérum, de plasma, d'urine, d'un prélèvement tissulaire ou de cellules isolées, et en particulier d'un milieu cellulaire. Dans le cas d'un échantillon issu d'un végétal, il peut s'agir d'un extrait de plante, de champignon ou d'algue, de cellules vivantes, et en particulier d'un milieu cellulaire. Il est également possible que l'échantillon comprenne directement le végétal. Dans le cas d'un échantillon issu d'un microorganisme, le microorganisme peut être une bactérie, un virus, un champignon ou une levure, et peut concerner également un microbiote. L'échantillon peut comprendre directement le microorganisme, ou bien un extrait de celui-ci ou encore du milieu de culture dans lequel le microorganisme a été incubé. Dans tous les cas, le prélèvement peut être utilisé tel quel, ou bien être soumis, avant mise en présence de la sonde, à une préparation de type enrichissement ou culture, bien connue de l'homme de l'art.

Dans le cas d'un échantillon provenant d'un animal, d'un végétal ou d'un microorganisme, l'invention concerne un procédé pour détecter la présence d'une glycosidase catalytiquement active comprenant les étapes de :
- mise en contact d'un échantillon suspecté de contenir ladite glycosidase avec un composé (I) selon l'invention, ledit,
- application de conditions appropriées pour permettre la formation d'un composé fluorescent, notamment sous la forme d'un précipité, par clivage de la liaison covalente entre O et R0, suivi d'un clivage de la liaison -C(O)OR1, suite à des réactions d'élimination et de cyclisation du tandem d'espaceurs, et
- analyse quantitative ou qualitative dudit précipité fluorescent.

L'analyse du composé ou précipité fluorescent peut comprendre :
- une étape d'exposition du précipité fluorescent à une source lumineuse capable de produire de la lumière à une longueur d'onde d'absorption du précipité fluorescent, et
- une étape de détection de la fluorescence du précipité résultante.

L'analyse peut en outre comprendre une étape, subséquente à l'étape de détection de la fluorescence, de tri des échantillons analysés basé sur le signal fourni par ledit précipité fluorescent. Les échantillons triés peuvent être des colonies de microorganismes séparées dans l'espace, tel que sur des boites de cultures microbiologiques. Les échantillons triés peuvent également être de petits objets, liquides, solides, gelatineux ou de composition hétérogène, contenant soit des biomolécules soit des colonies de microorganismes. Lorsque la détection est faite en parallèle sur plusieurs échantillons, le tri peut être effectué, par exemple, par déviation d'un flux d'échantillons mis en mouvement dans un dispositif permettant de les trier selon un signal optique, représentatif de la fluorescence émise, tel que cytomètre en flux ou dispositif de milli- ou micro-fluidique digitale.

La présente invention rend l'activité des glycosidases accessible par imagerie par fluorescence utilisant des fluorophores ESIPT. De manière avantageuse, aucun bruit de fond dû à une dégradation spontanée (c'est-à-dire en l'absence de glycosidase cible, en milieu physiologique) n'est observé. La sonde elle-même est peu ou n'est pas fluorescente, en particulier à la longueur d'onde d'émission du fluorophore ESIPT libre sur laquelle l'instrument de détection/imagerie est réglé. La sonde fonctionne ainsi dans le mode éteint/allumé et peut être utilisée pour le développement d'analyses avec une sensibilité maximale. Cette invention permet, en fonction du groupe R0 sélectionné, de cibler des glycosidases avec une sélectivité élevée pour des groupements glycosyles particuliers.

Les sondes selon l'invention sont attractives pour plusieurs applications à haute sensibilité dans les sciences de la vie, et notamment : (1) le criblage à haut rendement d'une activité glycosidase exprimée par des colonies bactériennes sur une plaque gélosée (analyse sur colonies) ; (2) la détection *in vitro* de glycosidase dans des liquides biologiques (hématologie et autres) ; (3) la visualisation d'une activité glycosidase au niveau d'une simple cellule en cytométrie de flux; (4) la détection de glycosidases subcellulaires dans des cellules cultivées (microscopie de fluorescence confocale) ; (5) la détection histochimique de glycosidase (à l'échelle tissulaire) ; et finalement (6) l'imagerie *in vivo* d'un animal entier.

Ainsi, les composés de formule (I), en tant que substrats de glycosidase selon la présente invention, ont un grand nombre d'applications potentielles. Les exemples de telles applications incluent la conception d'analyses sur colonies bactériennes. Celles-ci sont actuellement réalisées sur une plaque gélosée (boîte de Pétri) où jusqu'à 3 000 colonies peuvent être distinguées sans avoir à les séparer activement dans des compartiments séparés comme les puits contenus dans une plaque à puits multiples. Il est ainsi possible de (1) concevoir des tests sur échantillons cliniques permettant d'identifier parmi un ensemble de lignées bactériennes une lignée pathogène d'intérêt et (2) réaliser des tests parallèles massifs d'une banque de protéines de sa propre production exprimée par un hôte bactérien classique (souvent commercial). Cette collection de protéines peut bien entendu contenir une protéine d'intérêt particulier, par exemple une glycosidase ayant une sélectivité pour un groupement glycosyle spécifique, ou une glycosidase hydrolysant une liaison glycosidique non naturelle. Dans le domaine de l'évolution dirigée des glycosidases en général ou des enzymes en particulier, il existe une forte demande pour des analyses efficaces et sensibles pour cribler de très grands nombres de variants de protéines dépassant aisément 10⁶. L'application de la sonde selon l'invention peut être envisagée le plus aisément par dissolution dans la solution gélosée avant qu'elle soit versée dans la plaque où elle se gélifie. A titre d'alternative, les substrats sont incubés avec les colonies par immersion d'un filtre avant qu'il soit pressé sur les colonies. Le principal avantage auquel la sonde selon l'invention contribue pour une telle analyse sur colonies est la précipitation sur site du fluorophore ; une dilution du signal fluorescent par diffusion est donc très réduite, ce qui permet de plus grandes durées d'incubation et donc une plus grande sensibilité pour l'analyse. Le très grand déplacement de Stokes de la dichloro-HPQ (approximativement 140 nm) ou de tout analogue de la HPQ ne devrait pas rester mésestimé ; il contribue aussi à l'excellente sensibilité et la fluorescence émise est aisément distinguable de la fluorescence native qui pourrait provenir de l'échantillon biologique.

Les sondes selon l'invention peuvent servir aussi pour l'imagerie par fluorescence macroscopique, c'est-à-dire au niveau de l'organisme entier. Dans ce cas, la sonde pénétrera dans la paroi cellulaire pour atteindre l'activité d'intérêt.

Les exemples, en relation avec les figures annexées, permettent d'illustrer l'invention mais n'ont aucun caractère limitatif.
La **figure 1** est un schéma montrant le mécanisme de dégradation à partir des composés de formule (I) et faisant appel au tandem d'espaceurs.
La **figure 2** est une courbe de l'évolution de la fluorescence à l'état solide pour les sondes **13** et **27** des exemples 1 et 3, et pour la sonde **28** de l'art antérieur à 37 °C (concentration : 10 µM).
La **figure 3** représente les courbes de l'évolution de la fluorescence à l'état solide pour la sonde **21** de l'exemple 2 à 37 °C (concentrations : 0 µM, 5 µM, 10 µM, 25 µM et 50 µM).
La **figure 4** représente le signal lumineux témoignant de l'activité cellulase produite par un micro-organisme.
La **figure 5** est un graphe représentant la cinétique de détection d'activité cellulase dans un milieu de culture de microorganisme.

### Exemples

### Généralités

La chromatographie sur colonne a été réalisée sur gel de silice 60-mesh (40-63 µm). Les spectres de RMN de ¹H et de ¹³C ont été enregistrés à 300 MHz et à 75 ou 125 MHz, respectivement, dans le chloroforme deutéré, le DMSO deutéré ou le méthanol deutéré. Les déplacements chimiques (δ) sont indiqués en ppm et notés en référence au tétraméthylsilane ou d'après des signaux résiduels du solvant ; les abréviations s = singulet, d = doublet, t = triplet, m = multiplet, b = large sont utilisées. Les constantes de couplage de RMN (*J*) sont indiquées en Hertz. Les analyses par fluorescence ont été réalisées dans des plaques en polypropylène noir à 96 puits (Corning Costar, Corning Inc.), et enregistrées sur un fluorimètre à microplaques (EnSpire plate reader de Perkin Elmer). Sauf quand cela est spécifié, les produits chimiques ont été achetés avec la qualité de réactif analytique et utilisés sans autre purification.

Le DCM sec commercial a été séché et purifié par passage sur colonne d'alumine activée sous argon (GT S100 Solvant Station System). La TEA a été distillée à partir de l'hydrure de calcium et conservée sur des pastilles de KOH. Les autres réactifs notés secs ont été séchés sur tamis moléculaires. Si le contraire n'est pas indiqué, toutes les réactions ont été conduites sous atmosphère d'air avec des solvants et réactifs commerciaux, sans séchage ou purification supplémentaire. De l'eau millipore obtenue à partir d'un système de purification Elga Purelab a été utilisée dans toutes les expériences.

Les abréviations suivantes sont utilisées :
DIPEA = diisopropyléthylamine
TEA = triéthylamine
py = pyridine
DCM = dichlorométhane
Rdt = rendement
DMSO = diméthylsulfoxyde
TFA = acide trifluoroacétique
rt = température ambiante

### Exemple 1

Le composé **13** est préparé comme décrit sur le **Schéma 1** suivant.

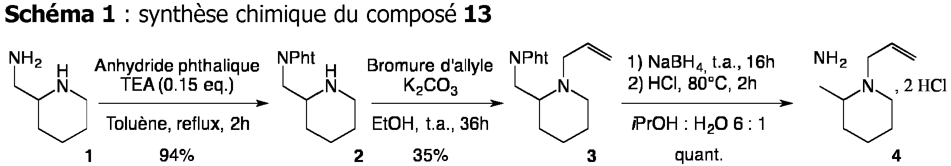

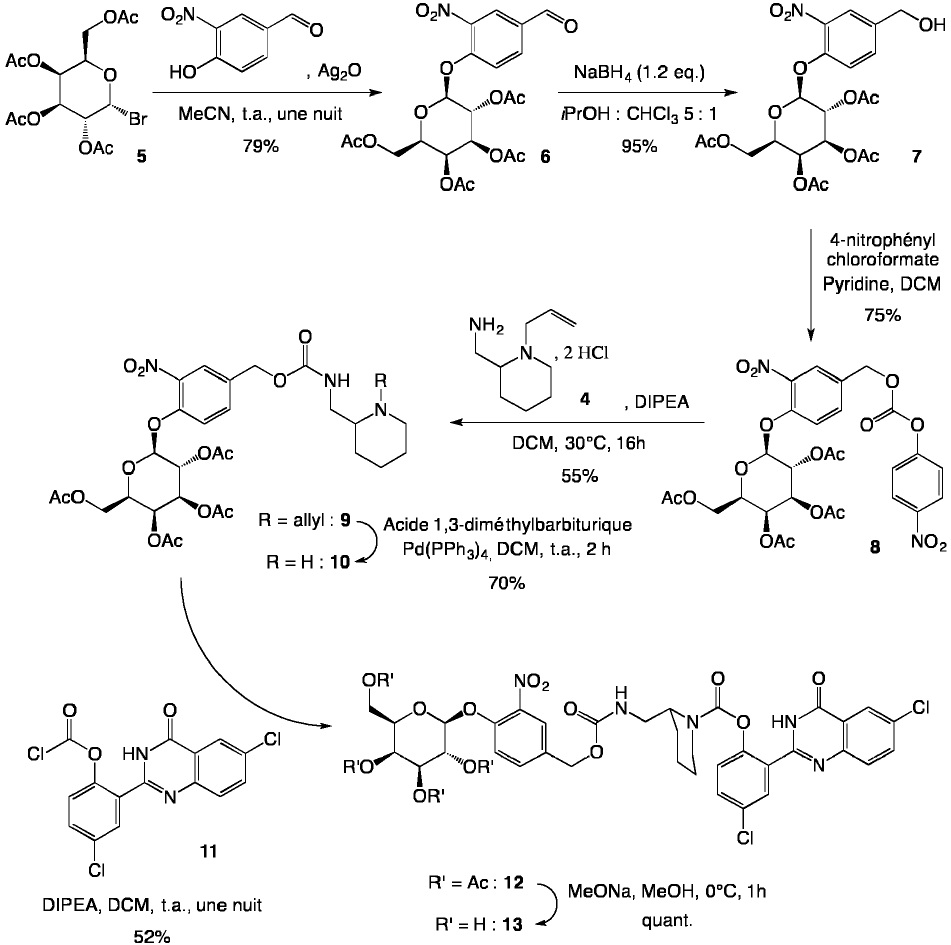

### Préparation du composé 2 :

A une solution de 2-aminométhylpipéridine **1** (3.0 g, 26.3 mmol, 1.0 eq.) dans 100 mL de toluène a été ajouté petit à petit de l'anhydride phtalique (3.89 g, 26.3 mmol, 1.0 eq.) et goutte à goutte de la triéthylamine (550 µL, 3.95 mmol, 0.15 eq.). Le mélange a ensuite été chauffé à reflux pendant 2h à l'aide d'un appareil Dean-Stark. Ensuite, le mélange a été filtré et le solvant a été évaporé sous pression réduite. Le composé **2** (6.605 g, 24.7 mmol, rdt: 94%) a été obtenu sous forme de solide jaune pâle et utilisé sans purification.

¹H-NMR (300 MHz, CDCl₃): *δ* (ppm) = 7.89-7.82 (m, 2H), 7.75-7.68 (m, 2H), 3.68 (d, *J* = 4 Hz, 2H), 3.13-3.05 (m, 1H), 2.98-2.88 (m, 1H), 2.64-2.54 (m, 1H), 1.87-1.78 (m, 1H), 1.76-1.68 (m, 1H), 1.63-1.54 (m, 1H), 1.45-1.34 (m, 2H), 1.30-1.15 (m, 1H). ¹³C-NMR (75 MHz, CDCl₃): *δ* (ppm) = 168.4, 133.7, 131.9, 123.0, 55.5, 46.4, 43.4, 30.6, 26.1, 24.1.

HRMS: ESI: [M+H]⁺ m/z trouvé 245.1290, calc. 245.1290

### Préparation du composé 3 :

A une solution de composé **2** (6.605 g, 24.7 mmol, 1.0 eq.) dans 80 mL d'éthanol refroidi dans un bain de glace, du carbonate de potassium (4.36 g, 31.6 mmol, 1.3 eq.), de l'iodure de tétra-n-butylammonium (912 mg, 2.47 mmol, 0.10 eq.) et du bromure d'allyle (2.73 mL, 31.6 mmol, 1.3 eq.) ont été ajoutés. Le bain de glace a été enlevé et le mélange a été agité pendant 36 h. A la fin de la réaction, le mélange a été filtré sur Célite et évaporé sous pression réduite. L'huile résiduelle a été dissolute dans EtOAc et une solution aqueuse saturée de NH₄Cl est ajoutée. Les deux phases ont été séparées et la phase organique a été lavée deux fois avec une solution aqueuse saturée de NH₄Cl. Les phases aqueuses combinées ont été extraites 3 fois avec EtOAc. Les phases organiques combinées ont été séchées avec Na₂SO₄, filtrées et concentrées sous pression réduite. Le produit brut a été purifié par colonne chromatographique sur gel de silice (DCM pur, puis DCM : MeOH : Et₃N / 99:0.5:0.5 / v:v:v) pour obtenir le composé **3** sous forme d'huile jaune qui cristallise (2.24 g, 7.89 mmol, yield: 35%).

¹H-NMR (300 MHz, CDCl₃): *δ* (ppm) = 7.84-7.78 (m, 2H), 7.72-7.65 (m, 2H), 5.95-5.81 (m, 1H), 5.22-5.08 (m, 2H), 3.93 (dd, 1H, *J* = 13 Hz, J = 5 Hz), 3.73 (dd, 1H, *J* = 13 Hz, *J* = 8 Hz), 3.42 (dd, 1H, *J* = 14 Hz, *J* = 6 Hz), 3.21 (dd, 1H, J = 14 Hz, *J* = 6 Hz), 2.88-2.76 (m, 2H), 2.37-2.28 (m, 2H), 1.74-1.47 (m, 4H), 1.40-1.25 (m, 2H).

¹³C-NMR (75 MHz, CDCl₃): *δ*(ppm) = 168.3, 135.5, 133.8, 132.0, 123.0, 117.2, 57.6, 57.3, 50.3, 38.4, 28.1, 24.7, 22.0.

HRMS: ESI: [M+H]⁺ m/z trouvé 285.1595, calc. 285.1603

### Préparation du composé 4 :

A une solution de **3** (1.312 g, 4.6 mmol, 1.0 eq.) dans *i*PrOH : H₂O / 6:1 / v:v (50 mL) refroidie dans de la glace a été ajouté petit à petit du borohydrure de sodium (874 mg, 23 mmol, 5.0 eq.). Après avoir agité à température ambiante pendant une nuit, le pH a été acidifié à pH = 1 à l'aide d'une solution aqueuse de HCl à 37%. Le mélange a été filtré et ensuite chauffé à 80°C pendant 2 h. *i*PrOH a été évaporé sous pression réduite et la solution aqueuse résultante a été lavée 5 fois avec de l'éther diéthylique et ensuite lyophilisé. Le composé **4** a été obtenu sous forme de poudre blanche (1.045 g, 4.6 mmol, rdt quantitatif).

RMN du produit basique (1-allyl-2-(aminométhyl)pipéridine) : ¹H-NMR (300 MHz, CDCl₃): *δ* (ppm) = 5.99-5.86 (m, 1H), 5.24-5.13 (m, 2H), 3.41 (ddt, 1H, *J* = 14 Hz, *J* = 5.7 Hz, *J* = 1.5 Hz), 3.04-2.90 (m, 3H), 2.74 (dd, 1H, *J* = 13 Hz, *J* = 3.3 Hz), 2.21 (tt, 2H, J = 9.6 Hz, J = 3.3 Hz), 1.80-1.71 (m, 1H), 1.68-1.43 (m, 2H), 1.39-1.25 (m, 3H).

¹³C-NMR (75 MHz, CDCl₃): *δ* (ppm) = 134.93, 116.87, 61.47, 56.23, 51.93, 42.97, 28.28, 24.95, 23.66.

HRMS: ESI: [M+H]⁺ m/z trouvé 155.1543, calc. 155.1548

### Préparation du composé 6 :

De l'acétobromogalactose **5** (700 mg, 1.70 mmol, 1.0 eq.), du 4-hydroxy-3-nitrobenzaldéhyde (313 mg, 1.87 mmol, 1.1 eq.) et de l'Ag₂O (1.300 g, 5.61 mmol, 3.3 eq.) ont été dissouts dans de l'acétonitrile et agités pendant une nuit à température ambiante. Le mélange réactionnel a ensuite été filtré sur Célite et concentré sous pression réduite. L'huile résultante a été purifiée par colonne chromatographique sur gel de silice (éther de pétrole : acétate d'éthyle / 4:6 / v:v) pour obtenir le composé **6** sous forme de solide jaune pâle (669 mg, 1.34 mmol, rdt : 79%).

¹H-NMR (300 MHz, DMSO-*d*₆): *δ* (ppm) = 9.98 (s, 1H), 8.45 (d, *J* = 2 Hz, 1H), 8.26 (dd, *J* = 9 Hz, *J* = 2 Hz, 1H), 7.60 (d, *J* = 9 Hz, 1H), 5.80 (d, *J* = 8 Hz, 1H), 5.40 (bs, 1H), 5.30-5.27 (m, 2H), 4.55 (td, *J* = 6 Hz, *J* = 1 Hz, 1H), 4.15 (d, *J* = 6 Hz, 2H), 2.16 (s, 3H), 2.05 (s, 3H), 2.04 (s, 3H), 1.96 (s, 3H).

¹³C-NMR (75 MHz, CDCl₃): *δ* (ppm) = 188.55, 170.24, 170.07, 169.16, 153.45, 141.25, 133.96, 131.48, 126.84, 118.80, 100.09, 71.82, 70.35, 67.59, 66.58, 61.36, 20.66, 20.61, 20.59, 20.55. HRMS: ESI: [M+Na]⁺ m/z trouvé 520.1052, calc. 520.1067

### Préparation du composé 7 :

A une solution de composé **6** (636 mg, 1.28 mmol, 1.0 eq.) dans CHCl₃ : *i*PrOH 5:1 v:v (12 mL) dans un bain de glace a été ajouté du borohydrure de sodium (53 mg, 1.41 mmol, 1.1 eq.). La réaction a été agitée pendant 1h et arrêtée par ajout d'une solution aqueuse saturée de NH₄Cl. Après 5 minutes d'agitation, les phases sont séparées et la phase aqueuse a été extraite 2 fois avec du dichlorométhane. Les phases organiques combinées ont été séchées avec Na₂SO₄, filtrées et évaporées sous pression réduite pour donner le composé **7** sous forme de poudre blanche
(605 mg, 1.22 mmol, rdt : 95%) qui a été utiliées dans l'étape suivante sans purification.

¹H-NMR (300 MHz, DMSO-*d*₆): *δ* (ppm) = 7.80 (d, *J* = 2 Hz, 1H), 7.63 (dd, *J* = 9 Hz, *J* = 2 Hz, 1H), 7.37 (d, *J* = 9 Hz, 1H), 5.56 (d, *J* = 7 Hz, 1H), 5,43 (t, *J* = 6 Hz, 1H), 5.37 (d, *J* = 3 Hz, 1H), 5.31-5.19 (m, 2H), 4.53-4.45 (m, 3H), 4.19-4.08 (m, 2H), 2.16 (s, 3H), 2.04 (s, 6H), 1.95 (s, 3H).

¹³C-NMR (125 MHz, DMSO-*d*₆): *δ* (ppm) = 170.54, 170.46, 170.13, 169.47, 147.70, 140.84, 138.90, 132.41, 122.80, 118.30, 99.34, 71.35, 70.54, 68.34, 67.72, 61.94, 61.87, 21.10, 20.98, 20.92.

HRMS: ESI: [M+Na]⁺ m/z trouvé 522.1209, calc. 522.1224

### Préparation du composé 8 :

A une solution de composé **7** (120 mg, 0.240 mmol, 1.0 eq.) dans du DCM sec (5mL) refroidie dans de la glace a été ajouté successivement du chloroformate de 4-nitrophényl (107 mg, 0.53 mmol, 2.2 eq.) et de la pyridine (48 µL, 0.60 mmol, 2.5 eq.) goutte à goutte. Le mélange réactionnel a été agité à 0°C pendant 30 min et à température ambiante pendant 2h. A la fin de la réaction, la réaction a été arrêtée à l'aide d'une solution aqueuse HCl 1 M et les phases ont été séparées. La phase organique a été lavée avec une solution de HCl 1 M, et les phases aqueuses combinées ont été extraites avec du DCM. Les phases organiques ont été séchées avec Na₂SO₄, filtrées et évaporées sous pression réduite. Le produit brut a été purifié par colonne chromatographique sur gel de silice (gradient éther de pétrole : acétate d'éthyle / 85:15 to 50:50 / v:v) pour obtenir le composé **7** sous forme de solide blanc (111 mg, 0.18 mmol, rdt : 75%).

¹H-NMR (300 MHz, CDCl₃): *δ* (ppm) = 8.32 (d, *J* = 9 Hz, 2H), 7.94 (d, *J* = 2 Hz, 1H), 7.64 (dd, *J* = 9 Hz, *J* = 2 Hz, 1H), 7.44-7.39 (m, 3H), 5.59 (dd, *J* = 10 Hz, *J* = 8 Hz, 1H), 5,52 (d, J = 3 Hz, 1H), 5.33 (s, 2H), 5.17-5.14 (m, 2H), 4.30 (dd, *J* = 11 Hz, *J* = 7 Hz, 1H), 4.23-4.14 (m, 2H), 2.23 (s, 3H), 2.17 (s, 3H), 2.11 (s, 3H), 2.06 (s, 3H).

¹³C-NMR (125 MHz, CDCl₃): *δ*= 171.0, 137.8, 135.1, 129.4, 128.9, 128.9, 128.8, 128.4, 127.3, 127.2, 80.1, 62.8, 54.2, 53.0, 49.7, 43.9, 41.0, 40.1, 28.5 ppm.

MS: ESI: [M+Na]⁺ m/z trouvé 687.1263, calc. 687.1286

### Préparation du composé 9 :

A une suspension de composé **4** (44 mg, 0.20 mmol, 1.3 eq.) dans du DCM (3 mL) a été ajouté le composé **8** (100 mg, 0.150 mmol, 1.0 eq.). Le mélange réactionnel est refroidi dans un bain de glace et de la DIPEA (81 µL, 0.47 mmol, 3.1 eq.) a été ajoutée. Après 5 minutes, le bain de glace est retiré et le mélange réactionnel est chauffé à 30°C pendant une nuit. Le mélange a ensuite été lavé avec des solutions aqueuses saturées de Na₂CO₃ et NaHCO₃, séchées avec Na₂SO₄, filtrées et évaporées sous pression réduite. Le produit brut a été purifié par colonne chromatographique sur gel de silice pour obtenir le composé **9** sous forme de solide blanc (56 mg, 0.083 mmol, rdt : 55%).

NMR: ¹H-NMR (300 MHz, CDCl₃): *δ* (ppm) = 7.82 (d, *J* = *2 Hz,* 1H), 7.53 (dd, *J* = *9 Hz, J* = *2 Hz,* 1H), 7.35 (d, *J* = *9 Hz,* 1H), 5.93-5.75 (m, 1H), 5.56 (dd, *J* = *10 Hz, J* = *8 Hz,* 1H), 5,48 (d, *J* = *3 Hz,* 1H), 5.32 (bs, 1H), 5.22-5.06 (m, 6H), 4.29-4.14 (m, 2H), 4.11-4.06 (m, 1H), 3.43-3.22 (m, 3H), 2.99-2.88 (m, 2H), 2.43-2.36 (m, 1H), 2.21 (s, 3H), 2.14 (s, 3H), 2.09 (s, 3H), 2.03 (s, 3H), 1.76-1.25 (m, 6H).

¹³C-NMR (125 MHz, CDCl₃): *δ*(ppm) = 170.31, 170.19, 170.13, 169.40, 156.26, 148.92, 141.30, 134.68, 133.19, 133.14, 124.59, 119.83, 117.75, 100.82, 71.47, 70.57, 67.85, 66.75, 64.74, 61.37, 58.30, 56.36, 51.99, 42.51, 28.97, 25.00, 23.73, 20.70, 20.67, 20.59.

HRMS: ESI: [M+H]⁺ m/z trouvé 680.2683, calc. 680.2667

### Préparation du composé 10 :

Une solution de composé **9** (20 mg, 0.029 mmol, 1.0 eq.) et d'acide 1,3-diméthylbarbiturique (37 mg, 0.24 mmol, 8.0 eq.) dans du DCM sec (3 mL) a été dégasée avec un flux d'argon. Ensuite, du palladium (0) tétrakis(triphénylphosphine) (0.6 mg, 0.0005 mmol, 2 mol %) a été ajouté. À la fin de la réaction, le mélange réactionnel a été évaporé à sec et purifié par colonne chromatographique sur gel de silice pour obtenir le composé **10** sous forme de solide blanc (13 mg, 0.020 mmol, rdt : 70%).

¹H-NMR (300 MHz, CDCl₃): *δ* (ppm) = 7.82 (d, *J* = 2 Hz, 1H), 7.52 (dd, *J* = 9 Hz, *J* = 2 Hz, 1H), 7.35 (d, *J* = 9 Hz, 1H), 5.56 (dd, *J* = 10 Hz, *J* = 8 Hz, 1H), 5.48 (d, *J* = 3 Hz, 1H), 5.32-5.26 (m, 1H), 5.14-5.06 (m, 4H), 4.30-4.15 (m, 2H), 4.11-4.06 (m, 1H), 3.29-3.20 (m, 1H), 3.11-3.00 (m, 2H), 2.72-2.58 (m, 2H), 2.20 (s, 3H), 2.14 (s, 3H), 2.09 (s, 3H), 2.03 (s, 3H), 1.87-1.77 (m, 1H), 1.68-1.59 (m, 2H), 1.42-1.34 (m, 2H), 1.31-1.26 (m, 1H). ¹³C-NMR (125 MHz, CDCl₃): *δ* (ppm) = 170.32, 170.19, 170.14, 169.40, 156.15, 148.92, 141.30, 133.14, 133.10, 124.59, 119.84, 100.82, 71.48, 70.57, 67.85, 66.73, 64.73, 61.36, 56.03, 46.88, 46.68, 30.26, 26.47, 24.26, 20.70, 20.67, 20.59.

HRMS: ESI: [M+H]⁺ m/z trouvé 640.2337, calc. 640.2354

### Préparation du composé 12:

A une suspension de composé **10** (13 mg, 0.020 mmol, 1.0 eq.) dans du DCM sec (2 mL) sous atmosphère d'argon et refroidie dans de la glace a été ajoutée goutte à goutte une solution de composé **11** (8 mg, 0.021 mmol, 1.05 eq.) et de DIPEA (10 µL, 0.060 mmol, 3.0 eq.). Après l'addition, le mélange réactionnel a été mélangé à 0°C pendant 30 min puis à température ambiante pendant une nuit. Le mélange réactionnel a ensuite été lavé 3 fois avec une solution aqueuse saturée de NaHCO₃ et la phase organique a été séchée avec Na₂SO₄, filtrée et évaporée sous pression réduite. Le produit brut a été purifié par colonne chromatographique sur gel de silice pour obtenir le composé **12** sous forme de poudre blanche (10 mg, 0.010 mmol, rdt : 52%).

¹H-NMR (300 MHz, CDCl₃): *δ* (ppm) = 10.41-10.29 (m, 1H), 8.24 (bs, 1H), 8.16-8.05 (m, 1H), 7.85-7.63 (m, 2.5H), 7.57-7.46 (m, 2H), 7.45-7.37 (m, 0.5H), 7.25-7.08 (m, 2H), 6.23-6.10 (m, 0.5H), 5.83-5.75 (m, 0.5H), 5.57 (dd, *J* = 10 Hz, *J* = 8 Hz, 1H), 5.49 (d, *J* = 3 Hz, 1H), 5.17-4.96 (m, 2H), 4.94-4.69 (m, 1H), 4.58 (bs, 1H), 4.33-3.99 (m, 4H), 3.79-3.63 (m, 1H), 3.39-3.01 (m, 2H), 2.21 (s, 3H), 2.16 (s, 3H), 2.09 (s, 3H), 2.04 (s, 3H), 1.82-1.24 (m, 6H).

¹³C-NMR (125 MHz, CDCl₃): *δ* (ppm) = 170.36, 170.20, 170.14, 169.39, 161.09, 156.42, 152.66, 149.05, 147.37, 140.98, 139.32, 135.29, 133.30, 132.64, 132.37, 130.70, 129.72, 127.87, 125.88, 125.39, 125.04, 124.39, 124.03, 122.30, 119.77, 114.09, 100.76, 71.43, 70.62, 67.84, 66.73, 64.44, 61.31, 40.84, 29.72, 29.40, 25.38, 22.72, 20.69, 20.60, 18.89.

HRMS: ESI: [M+H]⁺ m/z trouvé 972.2077, calc. 972.2109

### Préparation du composé 13 :

A une solution de composé **12** (10 mg, 0.010 mmol, 1.0 eq.) dans du méthanol sec (2 mL) dans un bain de glace a été ajouté du méthoxyde de sodium (1.1 mg, 0.020 mmol, 2.0 eq.). Le mélange réactionnel a été agité pendant 1 h à 0 °C. Ensuite, la réaction est arrêtée avec de la résine Dowex^{®}50×8-100, puis filtrée et concentrée sous pression réduite. Le produit **13** a été obtenu sous forme de résine blanche (8 mg, 0.010 mmol, rdt quantitatif). Une haute pureté a été obtenue à l'aide d'une HPLC préparative en phase inverse (isocratique, eau : acétonitrile 1:1 v:v avec 0.1% TFA) pour donner le composé **13** sous forme de poudre blanche.

¹H-NMR (300 MHz, CD₃OD): *δ* (ppm) = 8.07 (m, 1H), 7.73-7.69 (m, 2H), 7.67-7.60 (m, 2H), 7.50-7.31 (m, 2H), 7.31-7.23 (m, 1H), 7.13-7.05 (m, 1H), 5.02-4.93 (m, 1H), 4.93-4.82 (m, 2H), 4.51-4.40 (m, 1H), 4.13-4.03 (m, 1H), 3.83-3.78 (m, 1H), 3.77-3.71 (m, 1H), 3.68-3.58 (m, 3H), 3.53-3.44 (m, 2H), 3.12-3.01 (m, 1H), 2.94-2.83 (m, 1H), 1.59-1.25 (m, 6H).

¹³C -NMR (125 MHz, CD₃OD): *δ* (ppm) = 163.06, 158.56, 154.58, 152.42, 151.11, 149.16, 148.62, 141.78, 136.22, 134.26, 134.07, 132.97, 132.68, 132.09, 131.01, 130.43, 129.97, 126.45, 126.12, 125.42, 125.22, 123.48, 118.91, 103.10, 96.37, 77.36, 74.86, 71.96, 70.10, 65.76, 62.32, 53.19, 52.68, 41.16, 27.00, 26.27, 19.86.

HRMS: ESI: m/z [M+H]⁺ trouvé: 804.1671 calc. 804.1687

### Exemple 2

Le composé **21** est préparé comme décrit sur le **Schéma 2** suivant.

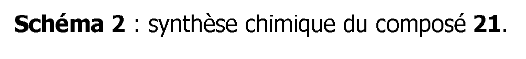

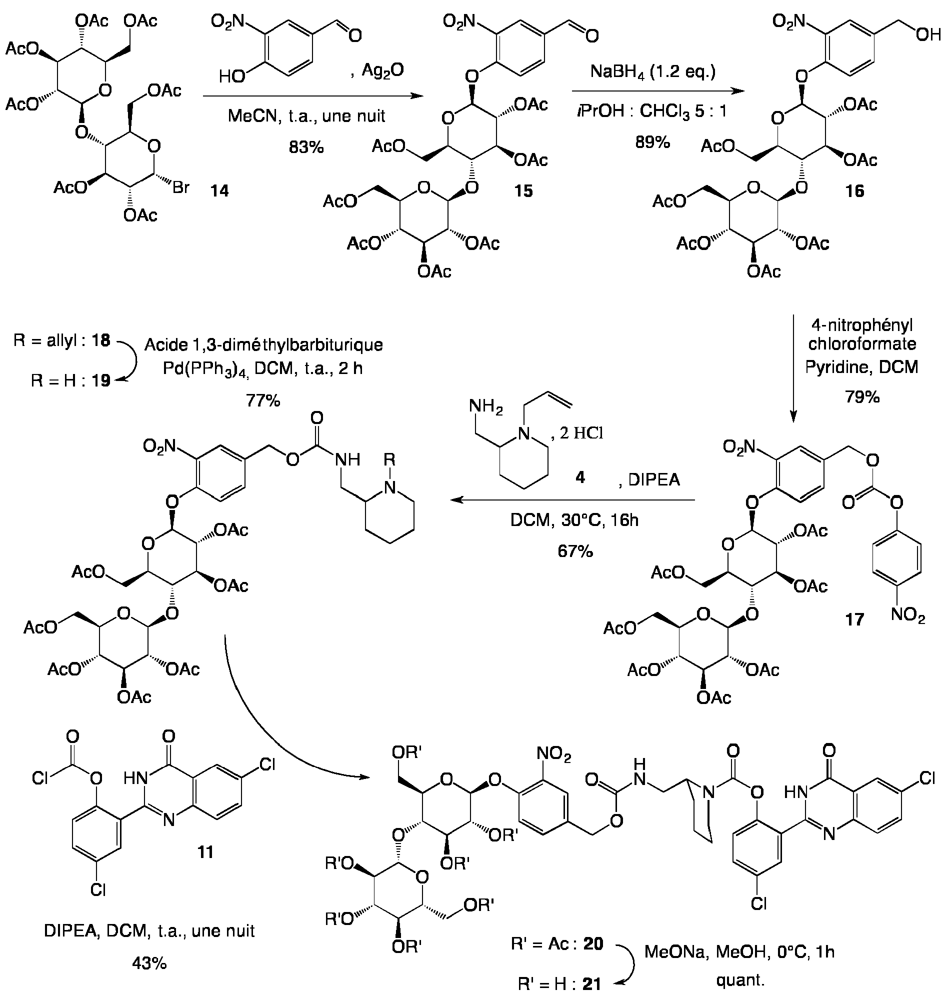

### Préparation du composé 15 :

Une procédure analogue à celle emloyée pour la préparation du composé **6** a été utilisée à partir de l'acétobromo-D-cellobiose **14** (2 g, 2.86 mmol, 1.0 eq.), de la 4-hydroxy-3-nitrobenzaldéhyde (478 mg, 2.86 mmol, 1.0 eq.) et de AgzO (729 mg, 3.15 mmol, 1.1 eq.) pour obtenir le composé **15** sous forme de solide jaune pâle (1.864 g, 2.37 mmol, rdt : 83%).

¹H-NMR (300 MHz, CDCl₃): *δ* (ppm) = 9.98 (s, 1H), 8.30 (d, *J* = 2 Hz, 1H), 8.07 (dd, *J* = 9 Hz, *J* = 2 Hz, 1H), 7.43 (d, *J* = 9 Hz, 1H), 5.35-5.05 (m, 5H), 4.95 (t, *J* = 8 Hz, 1H), 4.64-4.57 (m, 2H), 4.38 (dd, *J* = 13 Hz, *J* = 4 Hz, 1H), 4.16-3.98 (m, 3H), 3.94-3.87 (m, 1H), 3.74-3.67 (m, 1H), 2.12 (s, 3H), 2.11 (s, 3H), 2.08 (s, 3H), 2.04 (s, 3H), 2.03 (s, 3H), 2.02 (s, 3H), 2.00 (s, 3H).

¹³C-NMR (125 MHz, CDCl₃): *δ* (ppm) = 189.23, 170.88, 170.54, 170.46, 170.11, 169.71, 169.68, 169.47, 153.68, 141.40, 134.58, 131.65, 127.10, 118.64, 101.22, 98.98, 76.24, 73.60, 73.19, 72.39, 71.93, 70.97, 68.11, 61.91, 21.02, 20.88, 20.86.

HRMS: ESI: [M+Na]⁺ m/z trouvé 808.1874, calc. 808.1912

### Préparation du composé 16 :

Une procédure analogue à celle emloyée pour la préparation du composé **7** a été employée à partir du composé **15** (650 mg, 0.83 mmol, 1.0 eq.) et de NaBH₄ (34 mg, 0.91 mmol, 1.1 eq.) pour obtenir le composé **16** sous forme de poudre blanche (580 mg, 0.74 mmol, rdt : 89%), qui a été utilisé sans purification dans la réaction suivante.

¹H-NMR (300 MHz, CDCl₃): *δ* (ppm) = 7.82 (d, *J* = 2 Hz, 1H), 7.54 (dd, *J* = 9 Hz, *J* = 2 Hz, 1H), 7.32 (d, *J* = 9 Hz, 1H), 5.31-5.06 (m, 5H), 4.97 (t, *J* = 8 Hz, 1H), 4.74 (d, *J* = 6 Hz, 1H), 4.64-4.57 (m, 2H), 4.40 (dd, *J* = 13 Hz, *J* = 4 Hz, 1H), 4.15-4.07 (m, 2H), 4.01-3.95 (m, 1H), 3.84-3.78 (m, 1H), 3.74-3.68 (m, 1H), 2.14 (s, 3H), 2.12 (s, 3H), 2.09 (s, 3H), 2.08 (s, 3H), 2.06 (s, 3H), 2.04 (s, 3H), 2.01 (s, 3H), 1.87 (t, *J* = 6 Hz, 1H).

¹³C-NMR (125 MHz, CDCl₃): *δ* (ppm) = 170.48, 170.20, 170.12, 169.79, 169.54, 169.32, 169.11, 148.15, 141.10, 137.49, 131.78, 123.00, 119.23, 100.71, 99.61, 76.01, 73.04, 72.86, 72.23, 71.95, 71.60, 70.85, 67.83, 63.04, 61.63, 61.57, 20.60, 20.54, 20.41.

HRMS: ESI: [M+Na]⁺ m/z trouvé 810.2022, calc. 810.2069

### Préparation du composé 17 :

Une procédure analogue à celle emloyée pour la préparation du composé **8** a été utilisée à partir du composé **16** (400 mg, 0.51 mmol, 1.0 eq.), du chloroformate de 4-nitrophényle (225 mg, 1.07 mmol, 2.2 eq.) et de pyridine (102 µL, 1.27 mmol, 2.5 eq.) pour obtenir le composé **17** sous forme de poudre blanche (380 mg, 0.40 mmol, rdt : 79%).

¹H-NMR (300 MHz, CDCl₃): *δ* (ppm) = 8.32 (d, *J* = 9 Hz, 2H), 7.92 (d, *J* = 2 Hz, 1H), 7.63 (dd, *J* = 9 Hz, *J* = 2 Hz, 1H), 7.41 (d, *J* = 9 Hz, 2H), 7.35 (d, *J* = 9 Hz, 2H), 5.32-5.07 (m, 5H), 4.97 (t, *J* = 8 Hz, 1H), 4.67-4.58 (m, 2H), 4.64-4.57 (m, 2H), 4.40 (dd, *J* = 13 Hz, *J* = 4 Hz, 1H), 4.15-4.06 (m, 2H), 4.03-3.97 (m, 1H), 3.87-3.81 (m, 1H), 3.74-3.68 (m, 1H), 2.14 (s, 3H), 2.12 (s, 3H), 2.09 (s, 3H), 2.08 (s, 3H), 2.06 (s, 3H), 2.04 (s, 3H), 2.01 (s, 3H).

¹³C-NMR (75 MHz, CDCl₃): *δ* (ppm) = 170.51, 170.07, 170.05, 169.68, 169.33, 169.28, 169.03, 155.27, 152.18, 149.47, 145.42, 140.92, 133.94, 130.03, 125.35, 125.29, 121.71, 119.05, 100.75, 99.22, 75.98, 73.09, 72.80, 72.09, 71.91, 71.54, 70.70, 68.81, 67.74, 61.56, 61.53, 20.60, 20.55, 20.42.

HRMS: ESI: [M+Na]⁺ m/z trouvé 975.2103, calc. 975.2131

### Préparation du composé 18 :

Une procédure analogue à celle emloyée pour la préparation du composé **9** a été utilisée à partir des composés **17** (100 mg, 0.11 mmol, 1.0 eq.), **4** (30 mg, 0.20 mmol, 1.3 eq.) et de DIPEA (40 µL, 0.23 mmol, 2.1 eq.) pour obtenir le composé **18** sous forme de solide blanc (68 mg, 0.070 mmol, rdt : 67%).

¹H-NMR (300 MHz, CDCl₃): *δ* (ppm) = 7.82 (d, *J* = 2 Hz, 1H), 7.53 (dd, *J* = 9 Hz, *J* = 2 Hz, 1H), 7.28 (d, *J* = 9 Hz, 1H), 5.94-5.80 (m, 1H), 5.44-5.06 (m, 9H), 4.96 (t, *J* = 8 Hz, 1H), 4.64-4.56 (m, 2H), 4.40 (dd, *J* = 13 Hz, *J* = 4 Hz, 1H), 4.15-4.06 (m, 2H), 4.01-3.95 (m, 1H), 3.84-3.78 (m, 1H), 3.73-3.67 (m, 1H), 3.44-3.24 (m, 3H), 3.05-2.91 (m, 2H), 2.42 (bs, 1H), 2.22 (t, *J* = 11 Hz, 1H), 2.13 (s, 3H), 2.12 (s, 3H), 2.08 (s, 6H), 2.05 (s, 3H), 2.03 (s, 3H), 2.01 (s, 3H), 1.77-1.26 (m, 6H).

¹³C-NMR (75 MHz, CDCl₃): *δ* (ppm) = 170.49, 170.18, 170.12, 169.72, 169.47, 169.29, 169.02, 156.20, 148.73, 141.14, 134.66, 133.19, 132.93, 124.56, 119.18, 117.69, 100.80, 99.37, 75.94, 73.01, 72.86, 72.18, 72.06, 71.59, 70.84, 67.74, 64.64, 61.51, 61.48, 58.28, 56.31, 51.93, 42.47, 28.93, 24.94, 23.67, 20.69, 20.64, 20.54, 20.52.

HRMS: ESI: [M+H]⁺ m/z trouvé 968.3545, calc. 968.3512

### Préparation du composé 19 :

Une procédure analogue à celle emloyée pour la préparation du composé **10** a été utilisée à partir du composé **18** (68 mg, 0.070 mmol, 1.0 eq.), d'acide 1,3-diméthylbarbiturique (86 mg, 0.56 mmol, 8.0 eq.) et de palladium(0) tétrakis(triphénylphosphine) (1.6 mg, 0.0014 mmol, 2 mol %) pour obtenir le composé **19** sous forme de solide blanc (49 mg, 0.053 mmol, rdt : 77%).

¹H-NMR (300 MHz, CDCl₃): *δ* (ppm) = 7.79 (d, *J* = 2 Hz, 1H), 7.51 (dd, *J* = 9 Hz, *J* = 2 Hz, 1H), 7.27 (d, *J* = 9 Hz, 1H), 5.49-5.42 (m, 1H), 5.29-5.04 (m, 7H), 4.95 (t, *J* = 8 Hz, 1H), 4.63-4.55 (m, 2H), 4.39 (dd, *J* = 13 Hz, *J* = 4 Hz, 1H), 4.14-4.04 (m, 2H), 4.00-3.93 (m, 1H), 3.84-3.78 (m, 1H), 3.73-3.67 (m, 1H), 3.30-3.21 (m, 1H), 3.12-3.02 (m, 2H), 2.70-2.62 (m, 2H), 2.13 (s, 3H), 2.12 (s, 3H), 2.08 (s, 6H), 2.05 (s, 3H), 2.03 (s, 3H), 2.01 (s, 3H), 1.85-1.12 (m, 6H).

¹³C-NMR (75 MHz, CDCl₃): *δ* (ppm) = 170.50, 170.20, 170.12, 169.72, 169.48, 169.30, 169.02, 156.10, 148.74, 141.15, 133.18, 132.84, 124.56, 119.20, 100.82, 99.36, 75.93, 73.02, 72.87, 72.19, 72.08, 71.60, 70.85, 67.75, 64.68, 61.53, 61.47, 56.00, 46.83, 46.63, 30.22, 26.42, 24.22, 20.70, 20.66, 20.55, 20.53.

HRMS: ESI: [M+H]⁺ m/z trouvé 928.3230, calc. 928.3199

### Préparation du composé 20 :

Une procédure analogue à celle emloyée pour la préparation du composé **12** a été utilisée à partir des composés **19** (49 mg, 0.053 mmol, 1.0 eq.), **11** (21 mg, 0.054 mmol, 1.05 eq.) et de DIPEA (28 µL, 0.16 mmol, 3.0 eq.) pour obtenir le composé **20** sous forme de poudre blanche (29 mg, 0.023 mmol, rdt : 43%).

¹H-NMR (300 MHz, CDCl₃): *δ* (ppm) = 10.52 (bs, 1H), 8.21 (bs, 1H), 8.10-7.97 (m, 1H), 7.76-7.64 (m, 2.5H), 7.56 (m, 0.5H), 7.50-7.43 (m, 2H), 7.18-7.09 (m, 2H), 6.17-6.07 (m, 0.5H), 5.76 (bs, 0.5H), 5.30-5.04 (m, 5H), 4.95 (t, *J* = 8 Hz, 1H), 4.91-4.72 (m, 2H), 4.63-4.48 (m, 3H), 4.40 (dd, *J* = 13 Hz, *J* = 4 Hz, 1H), 4.19-4.04 (m, 3H), 4.00-3.93 (m, 1H), 3.83-3.57 (m, 3H), 3.37-3.18 (m, 1.5H), 3.11-2.98 (m, 0.5H), 2.12 (s, 3H), 2.11 (s, 3H), 2.08 (s, 3H), 2.06 (s, 6H), 2.03 (s, 3H), 2.01 (s, 3H), 1.81-1.44 (m, 6H).

¹³C-NMR (75 MHz, CDCl₃): *δ* (ppm) = 170.52, 170.21, 170.18, 169.74, 169.50, 169.31, 169.04, 161.13, 156.33, 154.23, 152.63, 149.11, 147.57, 147.34, 140.89, 135.28, 133.22, 132.80, 132.24, 130.59, 129.54, 127.82, 126.91, 125.85, 125.24, 124.84, 124.17, 122.22, 119.11, 100.85, 99.30, 75.97, 72.98, 72.90, 72.24, 72.20, 72.08, 71.61, 70.85, 67.76, 64.47, 61.53, 61.48, 51.46, 40.79, 26.03, 25.27, 20.70, 20.67, 20.54, 18.84.

HRMS: ESI: [M+H]⁺ m/z trouvé 1260.2914, calc. 1260.2954

### Préparation du composé 21 :

Une procédure analogue à celle emloyée pour la préparation du composé **13** a été utilisée à partir du composé **20** (23 g, 0.018 mmol, 1.0 eq) et de méthoxyde de sodium (1.0 mg, 0.036 mmol, 2.0 eq.) pour obtenir le composé **21** sous forme de poudre blanche (17 mg, 0.017 mmol, rdt : 97%).

¹H-NMR (300 MHz, CD₃OD): *δ* (ppm) = 8.19 (bs, 1H), 7.85-7.81 (m, 2H), 7.79-7.72 (m, 2H), 7.62-7.43 (m, 2H), 7.40-7.33 (m, 1H), 7.27-7.17 (m, 2H), 5.14-5.04 (m, 1.5H), 4.99-4.94 (m, 1H), 4.62-4.52 (m, 0.5H), 4.47 (d, *J* = 8 Hz, 2H), 4.26-4.16 (m, 1H), 3.97-3.82 (m, 3H), 3.74-3.56 (m, 5H), 3.44-3.35 (m, 3.5H), 3.30-2.95 (m, 3H), 3.11-2.98 (m, 0.5H), 1.72-1.15 (m, 6H). ¹³C-NMR (75 MHz, CD₃OD): *δ* (ppm) = 161.65, 157.10, 153.33, 153.05, 150.98, 149.35, 148.93, 147.75, 147.23, 140.47, 134.82, 132.84, 132.67, 131.57, 130.77, 129.62, 129.05, 128.55, 125.05, 123.86, 122.08, 117.46, 103.16, 100.77, 78.53, 76.75, 76.50, 75.55, 74.94, 73.51, 73.00, 72.99, 69.98, 64.34, 61.05, 60.19, 51.29, 39.52, 25.73, 24.79, 18.46.

HRMS: ESI: [M+H]⁺ m/z trouvé: 966.2201, calc. 966.2215

### Exemple 3

Le composé **27** est préparé comme décrit sur le **Schéma** 3 suivant.

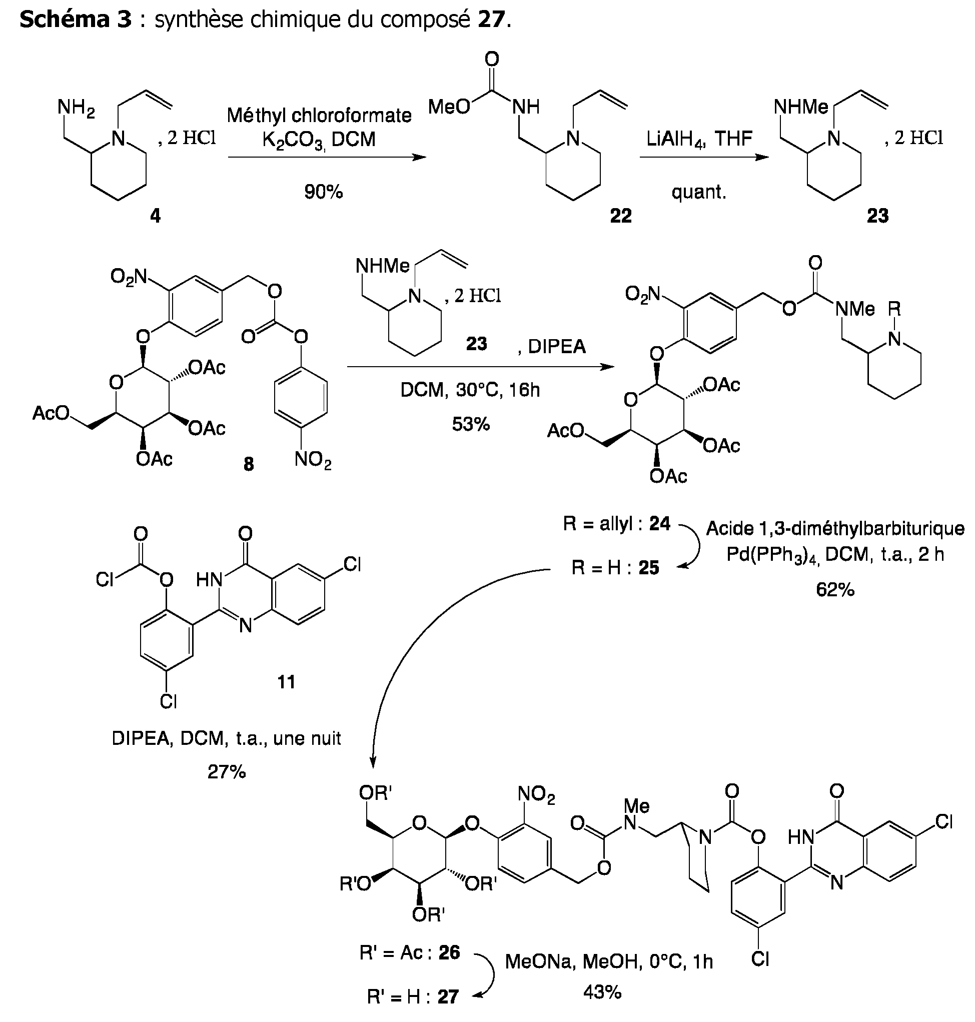

### Préparation du composé 22 :

A une solution de **3** (335 mg, 1.48 mmol, 1.0 eq.) dans 5 mL de dichlorométhane ont été ajoutés du carbonate de potassium (636 mg, 4.6 mmol, 3.1 eq.) et goutte à goutte du chloroformiate de méthyle (115 µL, 1.48 mmol, 1.0 eq.). Après 10 minutes d'agitation à température ambiante, le solvant a été évaporé sous pression réduite. Le produit brut a été purifié par colonne chromatographique sur gel de silice pour obtenir le composé **22** sous forme d'une huile jaune claire (282 mg, 1.33 mmol, rdt: 90%).

¹H-NMR (300 MHz, CDCl₃): *δ* (ppm) = 5.96-5.82 (m, 1H), 5.24-5.16 (m, 3H), 3.69 (s, 3H), 3.41-3.30 (m, 3H), 3.01-2.91 (m, 2H), 2.40 (m, 1H), 2.23-2.17 (m, 1H), 1.75-1.70 (m, 1H), 1.60-1.56 (m, 2H), 1.54-1.40 (m, 2H), 1.37-1.24 (m, 1H).

¹³C-NMR (75 MHz, CDCl₃): *δ*(ppm) = 157.34, 134.50, 117.71, 58.62, 56.29, 51.90, 42.31, 28.85, 24.92, 23.58.

HRMS: ESI: [M+H]⁺ m/z trouvé 213.1601, calc. 213.1603

### Préparation du composé 23 :

A une solution de tétrahydruroaluminate de lithium (2.64 mmol, 2.0 eq.) dans 5 mL de tétrahydrofurane, 22 (280 mg, 1.32 mmol, 1.0 eq.) a été ajouté goutte à goutte et le milieu a été agité à 40°C pendant une nuit. Le solvant a été évaporé et le produit **23** utilisé sans purification.

¹H-NMR (300 MHz, CDCl₃): *δ* (ppm) = 5.97-5.82 (m, 1H), 5.24-5.08 (m, 2H), 3.43-3.33 (m, 1H), 2.98-2.84 (m, 2H), 2.71-2.53 (m, 3H), 2.46-2.30 (m, 3H), 2.23-2.12 (m, 2H), 1.83-1.23 (m, 8H).

¹³C-NMR (75 MHz, CDCl₃): *δ* (ppm) = 127.43, 124.84, 61.65, 58.05, 55.71, 52.99, 52.19, 50.49, 48.26, 45.28, 34.14, 27.96, 26.19, 21.68, 21.43, 20.96, 20.02.

### Préparation du composé 24 :

Une procédure analogue à celle emloyée pour la préparation du composé **9** a été utilisée à partir des composés 8 (150 mg, 0.23 mmol, 1.0 eq.), **23** (100 mg, 0.42 mmol, 1.8 eq.) et de DIPEA (300 µL, 1.72 mmol, 7.6 eq.) pour obtenir le composé **24** sous forme de solide blanc (84 mg, 0.12 mmol, rdt : 53%).

¹H-NMR (500 MHz, CDCl₃): *δ* (ppm) = 7.75 (s, 1H), 7.48 (dd, *J* = 9 Hz, *J* = 2 Hz, 1H), 7.30 (d, *J* = 9 Hz, 1H), 5.90-5.72 (m, 1H), 5.50 (dd, *J* = 10 Hz, *J* = 8 Hz, 1H), 5,42 (d, *J* = 3 Hz, 1H), 5.14 (d, *J* = 6 Hz, 1H), 5.12-4.99 (m, 6H), 4.25-4.11 (m, 2H), 4.11-4.01 (m, 1H), 3.58-3.47 (m, 1H), 3.35-3.19 (m, 2H), 3.09-2.96 (m, 1H), 2.88 (d, *J* = 3 Hz, 3H), 2.81-2.71 (m, 1H), 2.68-2.58 (m, 1H), 2.14 (s, 3H), 2.08 (s, 3H), 2.02 (s, 3H), 1.97 (s, 3H), 1.70-1.36 (m, 6H).

¹³C-NMR (125 MHz, CDCl₃): *δ* (ppm) = 170.29, 170.18, 170.11, 169.38, 156.04, 148.92, 141.16, 135.08, 133.27, 133.23, 124.60, 119.66, 117.51, 100.69, 71.41, 70.53, 67.81, 66.74, 65.25, 61.36, 57.46, 57.10, 51.26, 49.88, 35.68, 30.92, 29.67, 28.20, 24.90, 22.60, 20.64.

LRMS: ESI: [M+H]⁺ m/z trouvé 694.2, calc. 694.2823.

### Préparation du composé 25 :

Une procédure analogue à celle emloyée pour la préparation du composé **10** a été utilisée à partir du composé **24** (84 mg, 0.12 mmol, 1.0 eq.), d'acide 1,3-diméthylbarbiturique (95 mg, 0.61 mmol, 5.0 eq.) et de palladium(0) tétrakis(triphénylphosphine) (1 mg, 0.0012 mmol, 1 mol %) pour obtenir le composé **25** sous forme de solide blanc (49 mg, 0.07 mmol, rdt : 62%).

¹H-NMR (500 MHz, CDCl₃): *δ* (ppm) = 7.78 (d, *J* = 9 Hz, 1H), 7.49 (d, *J* = 8 Hz, 1H), 7.30 (d, *J* = 9 Hz, 1H), 5.51 (dd, *J* = 10 Hz, *J* = 8 Hz, 1H), 5.43 (d, *J* = 3 Hz, 1H), 5.32-5.26 (m, 1H), 5.13-4.98 (m, 4H), 4.24-4.19 (m, 1H), 4.16-4.10 (m, 1H), 4.08-4.03 (m, 1H), 3.26-3.19 (m, 1H), 3.18-3.12 (m, 1H), 3.06-3.01 (m, 1H), 2.93 (d, *J* = 7 Hz, 3H), 2.82-2.68 (m, 1H), 2.61-2.51 (m, 1H), 2.15 (s, 3H), 2.09 (s, 3H), 2.04 (s, 3H), 1.98 (s, 3H), 1.79-1.75 (m, 1H), 1.61-1.50 (m, 2H), 1.42-1.20 (m, 3H).

¹³C-NMR (125 MHz, CDCl₃): *δ* (ppm) = 170.34, 170.22, 170.16, 169.42, 156.23, 148.93, 141.26, 133.30, 133.20, 124.59, 119.74, 100.77, 71.47, 70.57, 67.86, 66.77, 65.28, 61.39, 55.55, 55.12, 46.80, 36.00, 30.56, 26.30, 24.35, 20.72, 20.70, 20.62.

HRMS: ESI: [M+H]⁺ m/z trouvé 654.2484, calc. 654.2504

### Préparation du composé 26 :

Une procédure analogue à celle emloyée pour la préparation du composé **12** a été utilisée à partir des composés **25** (25 mg, 0.04 mmol, 1.0 eq.), **11** (21 mg, 0.04 mmol, 1.0 eq.) et de DIPEA (33 µL, 0.19 mmol, 5.0 eq.) pour obtenir le composé **26** sous forme de poudre blanche (10 mg, 0.01 mmol, rdt : 27%).

¹H-NMR (300 MHz, CDCl₃): *δ* (ppm) = 10.65-10.32 (m, 1H), 8.27-8.14 (m, 1H), 8.06-7.90 (m, 1H), 7.86-7.67 (m, 3H), 7.57-7.46 (m, 1H), 7.46-7.34 (m, 1H), 7.34-7.21 (m, 1H), 7.11-7.03 (m, 1H), 5.56-5.49 (m, 1H), 5.46 (br s, 1H), 5.13-4.98 (m, 2H), 4.98-4.67 (m, 1H), 4.55 (br s, 1H), 4.31-3.97 (m, 4H), 3.93-3.78 (m, 1H), 3.28-3.03 (m, 2H), 3.01-2.88 (m, 3H), 2.19 (s, 3H), 2.12 (s, 3H), 2.04 (s, 3H), 2.01 (s, 3H), 1.78-1.23 (m, 6H).

¹³C-NMR (75 MHz, CDCl₃): *δ* (ppm) = 170.41, 170.30, 170.25, 169.52, 160.53, 156.49, 153.10, 149.15, 147.52, 141.32, 139.29, 135.29, 133.31, 132.92, 132.28, 130.79, 129.79, 127.87, 126.09, 125.49, 125.13, 124.45, 124.38, 122.55, 119.83, 114.28, 100.84, 71.54, 70.67, 67.94, 66.82, 65.99, 61.42, 40.78, 29.83, 29.46, 26.68, 25.40, 20.78, 20.70, 20.54, 19.04.

HRMS: ESI: [M+H]⁺ m/z trouvé 986.2219, calc. 986.2260

### Préparation du composé 27 :

Une procédure analogue à celle emloyée pour la préparation du composé **13** a été utilisée à partir du composé **26** (10 g, 0.01 mmol, 1.0 eq) et de méthoxyde de sodium (2.0 mg, 0.04 mmol, 3.5 eq.) pour obtenir le composé **27** sous forme de poudre blanche (3.57 mg, 0.004 mmol, rdt : 43%).

Les spectres RMN sont trop peu résolus pour être attribués.

HRMS: ESI: [M+H]⁺ m/z trouvé: 818.1838, calc. 818.1838

### Exemple 4

Les sondes **13, 21** et **27** selon l'invention ont été évaluées par incubation avec l'enzyme cible, la β-galactosidase (EC 3.2.1.23 ; « b-gal » ; commercial) dans un milieu *in vitro* dans des microplaques à multi-puits conçues pour des lecteurs à fluorescence. Les sondes ont été évaluées selon les critères suivants :
- mise en évidence de l'intensité de fluorescence élevée générée par la présence de l'activité enzymatique (« allumé »),
- mise en évidence de l'absence totale (« éteint ») de fluorescence dans des échantillons qui ne contiennent pas l'enzyme cible (pas de fluorescence intrinsèque),
- mise en évidence de l'absence de toute dégradation hydrolytique de la sonde au cours du temps démontrant la robustesse de la sonde à pH 7 dans un milieu aqueux (pas de signal faussement positif),
- mise en évidence de la rapidité de réponse à la présence de l'activité enzymatique permettant d'atteindre rapidement un signal maximal,
- mise en évidence de la cinétique améliorée de la sonde à deux espaceurs,
- mise en évidence de la forte photo-stabilité du fluorophore solide généré sous irradiation prolongée par le lecteur de fluorescence.

Ces résultats ont été comparés avec ceux obtenus avec une sonde de l'art antérieur (composé I.1 de la demande WO 2014/020285) comportant un espaceur de type cyclisant (**28**) :

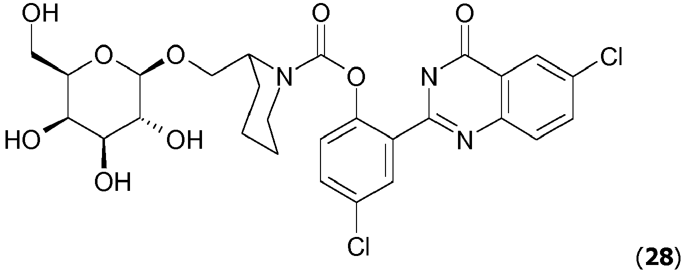

### Protocole de détection de la fluorescence :

Des solutions mères de sondes à 10 mM dans MeOH ont été diluées avec PBS (Dulbecco's Phosphate Buffer Saline, Invitrogen Corp.) pour obtenir des solutions avec des gammes de concentration allant de 50 µM à 1 mM. 10 µL de chacune de ces solutions ont été ajoutées à 80 µL de PBS dans une plaque noire à 96 puits, et chauffées à 37 °C avant addition de l'enzyme purifiée. Les concentrations finales de sonde étaient comprises dans la gamme allant de 5 µM à 100 µM. La plaque a ensuite été incubée à 37 °C (ou 25 °C) et la fluorescence a été mesurée au cours du temps par un lecteur de fluorescence (EnSpire, Perkin Elmer ; longueurs d'onde d'acquisition : *λₑₓ* = 355 nm, *λₑₘ*= 530 nm). Les courbes résultantes sont la moyenne de duplicats.

### Résultats :

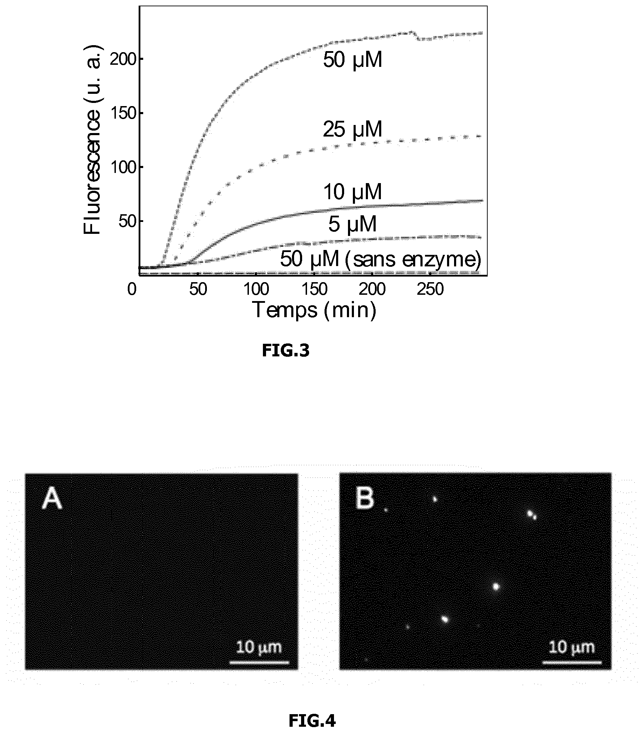

Les résultats obtenus sont présentés sur les **figures 2** et **3****.**

Comparé à la sonde **28,** la sonde **13** selon l'invention comportant un tandem d'espaceur éliminant/cyclisant permet de bénéficier d'une plus grande rapidité de réponse tout en conservant une haute stabilité de la sonde en l'absence de l'enzyme cible (absence de faux signal positif). Ainsi, dans les mêmes conditions de température, de pH et de concentration, la sonde **13,** basée sur un tandem d'espaceurs, a une réponse enzymatique 5 fois plus rapide que celle de la sonde **28** qui ne comporte qu'un seul espaceur. De plus, en l'absence d'enzyme, la sonde **13** est stable sur plus de 15h et ne génère aucune fluorescence mesurable.

Les sondes **21** et **27** selon l'invention permettent de bénéficier d'une plus grande rapidité de réponse tout en conservant une haute stabilité de la sonde en l'absence de l'enzyme cible (absence de faux signal positif).

La sonde **21** a été testée à différentes concentration : 5 µM, 10 µM, 25 µM et 50 µM. La fluorescence mesurée est proportionnelle à la concentration de la sonde. Une fluorescence peut être détectée dès une teneur de 5 µM de la sonde **21.**

### Exemple 5

Un surnageant de culture d'une souche de levure ne sécrétant pas (A) ou sécrétant (B) une β-glucosidase a été ajouté à la sonde **21** selon l'invention répondant à cette activité enzymatique. Pour cela, des cellules de levures portant un plasmide qui confère une résistance à l'hygromycine et portant ou non une cassette d'expression d'une beta-glucosidase sécrétée sont cultivées pendant 86h à 30°C dans 5 mL de milieu riche YPD (10 g de Bacto Peptone Difco, 10 g de Bacto Yeast Extract Difco, 20 g de Glucose, 20 g de Bacto Agar, qsp 1L d'eau distillée) contenant 200 µg/mL d'hygromycine. La culture est ensuite centrifugée à 4000 tr/min sur une centifugeuse Allegra 25R (Beckman/Coulter), dans un rotor oscillant (TS-5.1-500) à 20°C et 20 µL de surnageant sont prélevés et rajoutés à 180 µL d'une solution PBS1X contenant la sonde substrat à 50 µM. Le mélange est ensuite homogénéisé et incubé 30 minutes à 37°C. Puis, 10 µL de ce mélange sont déposés entre lame et lamelle de microscope et observés par microscopie à fluorescence avec un filtre d'excitation à 340 nm et un filtre d'émission à 525 nm.

Les photographies, représentées en **figure 4****,** sont obtenues au grossissement 100x à immersion sur un microscope Zeiss, AX10.

Les précipités fluorescents (points blancs) apparaissent distinctement ce qui permet d'envisager une utilisation par imagerie haut débit (segmentation et quantification automatisée).

### Exemple 6

La cinétique de détection d'activité cellulase dans un milieu de culture de microorganisme a été évaluée par lecture optique sur appareil MITHRAS LB 940 de l'activité enzymatique d'un surnageant de culture de cellules de levure qui sécrètent ('Surnageant') ou qui ne sécrètent pas ('Contrôle') la β-glucosydase.

Des cultures de levures sont menées comme dans le protocole décrit à l'exemple 5 jusqu'à l'obtention de 20 µL de surnageant. Ce surnageant est ensuite rajouté à 180 µL d'une solution PBS1X contenant la sonde **21** selon l'invention à 50 µM dans un puits de microplaques à fond opaque. La lecture du signal est faite au cours du temps sur un appareil MITHRAS LB 940, après excitation de la sonde à 340 nm et recueil de l'émission à 535 nm.

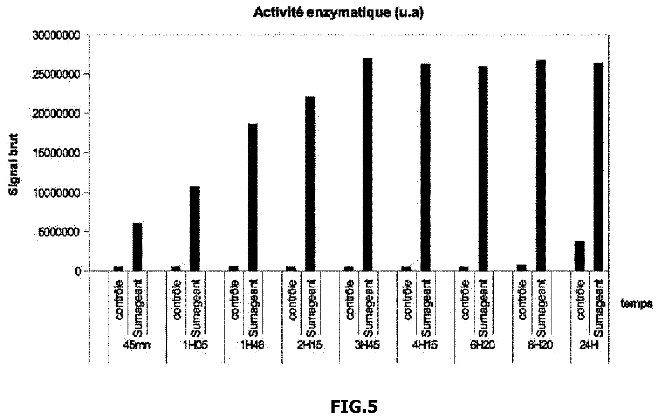

Les résultats, représentés en **figure 5****,** montrent que l'invention permet de détecter l'activité glycosidase sécrétée dans le surnageant dès 45 minutes d'incubation, que le signal est maximal après 3h45 d'incubation, et que le rapport signal sur bruit est alors d'environ 55.

## Revendications

1. Composés de formule (I) : 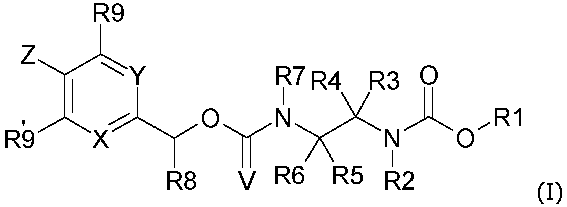 dans laquelle :
- R1 est tel que HOR1, obtenu après clivage de la liaison -C(O)-OR1 présente sur la formule (I), appartient à la classe des fluorophores conduisant à un transfert de proton intramoléculaire dans un état excité, nommés ESIPT, R1 étant un groupe aromatique avec -OR1 qui répond à la formule (A1) : 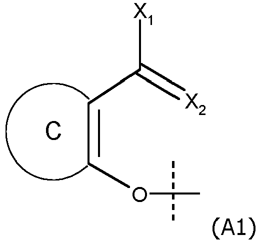 dans laquelle :
- soit X2 est un atome d'oxygène et X1 est un groupe -NH₂, -OH, -SH, (C1-C20)alkyle, (C5-C24)aryle, -O-(C1-C20)alkyle, -O-phényle, -NH-(C1-C20)alkyle ou -NH-phényle, -S-(C1-C20)alkyle ou -S-(C5-C24)aryle, lesdits groupes alkyle et phényle pouvant être substitués ou non substitués,
soit X2 représente un atome d'azote et est lié à X1 qui représente alors CH, O, S, N ou NH pour former un (C5-C24)hétéroaryle substitué ou non substitué, 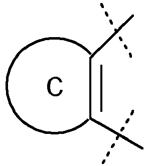 représente un (C5-C24)aryle ou un (C5-C24)hétéroaryle, substitué ou non substitué, par exemple choisi parmi les groupes phényle, naphthyle, et : 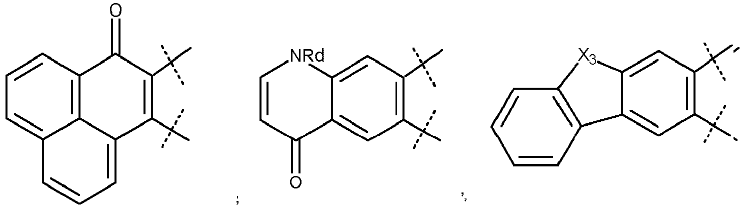
lesdits groupes pouvant être substitués ou non substitués,
- avec X3 qui représente S, O ou NRd et Rd qui représente un atome d'hydrogène ou un groupe (C1-C4)alkyle,
- R2, R3 et R4 sont définis comme suit :
∘ soit R2 est un (C1-C4)alkyle, R3 est un (C1-C4)alkyle ou un atome d'hydrogène, et R4 est un (C1-C4)alkyle,
∘ soit R3 est un (C1-C4)alkyle ou un atome d'hydrogène et R2 et R4 sont liés entre eux et forment avec les atomes de carbone et d'azote auxquels ils sont liés un hétérocycle aliphatique, cet hétérocycle pouvant être substitué par un groupement hydrosolubilisant,
∘ soit R2 est un (C1-C4)alkyle et R3 et R4 sont liés entre eux et forment avec l'atome de carbone auxquels ils sont liés un carbocycle aliphatique,
- R5 et R6 sont identiques ou différents et représentent indépendamment l'un de l'autre un atome d'hydrogène, un (C1-C4)alkyle, ou un (C5-C10)aryle,
- R7 est un atome d'hydrogène, ou un groupe choisi parmi les (C1-C4)alkyle et (C1-C4)alcoxy,
- R8 représente un atome d'hydrogène, ou un groupe (C1-C10)alkyle, substitué ou non substitué, ou un groupe -D1-D2-D3 avec :
∘ D1 représentant un groupe triazolyle ou -CH₂-triazolyle,
∘ D2 représentant un groupe (C1-C10)alkylène, (C1-C10)alcènylène, ou (C1-C10)alcynylène, lesdits groupes étant éventuellement interrompus par un ou plusieurs hétéroatomes choisis parmi O ou N, un groupe glycosyle divalent, un groupe -O-(CHR-CHR'-O)ₙ- ou -N-(CHR-CHR'-O)ₙ-, n étant un entier variant de 1 à 20, R et R', identiques ou différents, représentant H ou CH₃ à condition que R et R' ne soient pas simultanément CH₃, un acide aminé ou un peptide, ou une combinaison de ces groupes,
∘ D3 représentant un motif maléimidocaproyl, acide aminé peptide, acide folique, anticorps ou fragment d'anticorps lié à D2, par une fonction acide carboxylique qu'il comprend, de manière à former un lien ester ou amide,
- R9 et R'9, identiques ou différents, représentent un atome d'hydrogène, ou un groupe électro-attracteur tel qu'un atome d'halogène, ou un groupe choisi parmi -NO₂, -CN, ou un groupe choisi parmi -NH-C(O)-CH₂-Ab, avec Ab représentant un anticorps
- V représente un atome d'oxygène ou un atome de soufre,
- X, Y et Z sont tels que :
∘ soit X représente CR10, Y représente CR'10 et Z représente OR0,
∘ soit X représente CR10, Y représente CORO et Z représente R'10,
∘ soit X représente CR10, Y représente un atome d'azote et Z représente OR0,
∘ soit X représente un atome d'azote, Y représente CORO et Z représente R10
avec :
∘ R0 représentant un groupement glycosyle lié par son carbone anomérique au reste de la molécule de formule (I), et
∘ R10 et R'10, identiques ou différents, représentant un atome d'hydrogène ou un groupe électro-donneur tel qu'un (C1-C20)alkyle, un (C5-C24)aryle, ou un (C1-C20)alcoxy,
sous la forme d'un mélange d'isomères optiques selon toutes proportions, ou sous une forme enrichie en un isomère optique.

2. Composés (I) selon la revendication 1, **caractérisés en ce que** R3 est un atome d'hydrogène ou un groupe (C1-C4)alkyle, de préférence un atome d'hydrogène, et R2 et R4 sont liés entre eux et forment un enchainement -(CH₂)ₘ- avec m = 3, 4 ou 5.

3. Composés (I) selon la revendication 1, **caractérisés en ce que** R3 est un atome d'hydrogène ou un groupe (C1-C4)alkyle, de préférence un atome d'hydrogène, et R2 et R4 sont liés entre eux et forment un enchainement -CH₂CH₂-NR11-CH₂- dans le sens de R2 vers R4, R11 représentant un atome d'hydrogène ou -(L)n-GP avec n qui est égal à 0 ou 1, L un bras de liaison et GP un groupe hydrosolubilisant.

4. Composés (I) selon la revendication 1, **caractérisés en ce que** R2, R3 et R4, identiques ou différents, représentent un groupe (C1-C4)alkyle, par exemple méthyle ou éthyle.

5. Composés (I) selon l'une quelconque des revendications 1 à 4, caractérisés en ce que-OR1 est du type phénoxy, et correspond à l'une des structures (A2) ou (A3) suivantes : 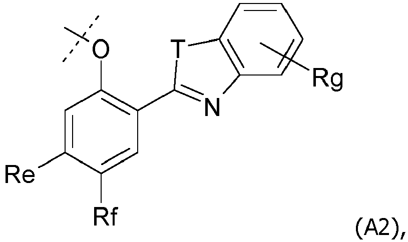 dans laquelle :
∘ T est -NH-C(O)-, -S-, -O-, -NH-, -N((C1-C20)alkyle)- ou -N((C5-C24)aryle)-,
∘ Re est un atome d'hydrogène ou un substituant carboné attracteur d'électrons comme -CN ou -COORh, avec Rh qui représente un groupe (C1-C4)alkyle, ou bien Re est -CONRiRj, avec Ri et Rj, identiques ou différents, qui représentent un atome d'hydrogène ou un groupe (C1-C4)alkyle, ou bien Re est -CF₃, ou un groupe 2-oxazolyle, 2-thiazolyle, 2-imidazolyle, 2-benzoimidazolyle, 4-pyrimidinon-2-yle ou quinazolinon-2-yle,
∘ Rf est un atome d'hydrogène, un atome de chlore, brome, iode ou fluor, - OH, -NH₂, -NRkRI, -NHRk ou -ORk, avec Rk et Rl identiques ou différents qui représentent chacun indépendamment un groupe (C1-C4)alkyle,
∘ ou bien Re et Rf sont liés entre eux pour former une chaîne hydrocarbonée comprenant 4 ou 5 chainons, saturée ou insaturée, substituée ou non substituée, éventuellement interrompue par un ou plusieurs hétéroatomes choisis parmi N, 5 et O,
∘ Rg est un atome d'hydrogène, Br, CI, I ou F, 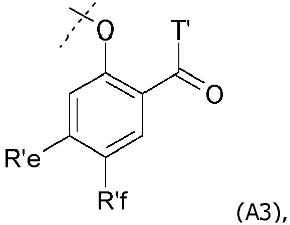 dans laquelle :
∘ T' est -NH₂, -OH, un groupe (C5-C24)aryle, un groupe (C1-C4)alkyle, -SH, -NHR'g, -OR'g, -NR'gRh' ou -SR'g, R'g et Rh', identiques ou différents, représentant un groupe (C1-C4)alkyle ou aryle,
∘ R'e est atome d'hydrogène ou un substituant carboné attracteur d'électrons comme -CN, ou -COOR'i, avec R'i qui représente un groupe (C1-C4)alkyle, ou R'e est -CON R'j R'k, avec R'j et R'k, identiques ou différents, qui représentent un atome d'hydrogène ou un groupe (C1-C4)alkyle, ou bien R'e est -CF₃, ou un groupe 2-oxazolyle, 2-thiazolyle, 2-imidazolyle, 2-benzoimidazolyle, 4-pyrimidinon-2-yle, ou quinazolinon-2-yle,
∘ R'f est un atome d'hydrogène, de chlore, de brome, d'iode ou de fluor, - OH, -NH₂, -NR'IR'm ou -OR'I, avec R'l et R'm, identiques ou différents, qui représentent un groupe (C1-C4)alkyle,
∘ ou bien R'e et R'f sont liés entre eux pour former une chaîne hydrocarbonée comprenant 4 ou 5 chainons, saturée ou insaturée, substituée ou non substituée, éventuellement interrompue par un ou plusieurs hétéroatomes choisis parmi N, 5 et O.

6. Composés (I) selon l'une quelconque des revendications 1 à 5, **caractérisés en ce que** R1 est un groupe aromatique avec -OR1 qui répond à la formule (A5) suivante :

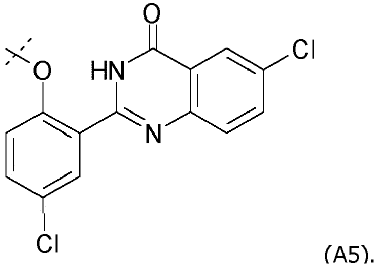

7. Composés (I) selon l'une quelconque des revendications 1 à 6, **caractérisés en ce que** R0 est un groupement mono-glycosylé lié par son carbone anomérique choisi parmi le galactosyle, le glucosyle, le mannosyle, le gulosyle, l'allosyle, l'altrosyle, l'idosyle, le talosyle, le fucosyle, le fructosyle, l'arabinosyle, le lyxosyle, le ribosyle, le xylosyle, le glucuronyle et le N-acétyl-hexosaminyle ou un groupement polyglycosylé constitué de plusieurs de ces groupements monoglycosylés identiques ou différents.

8. Composés (I) selon l'une quelconque des revendications 1 à 7 de formule (Ib) : 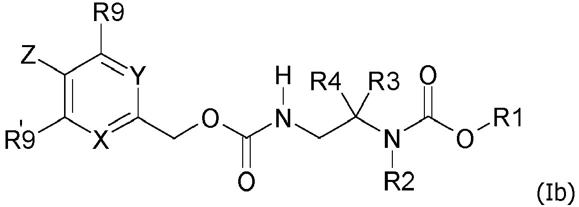 où R1, R2, R3, R4, R9, R'9, X, Y et Z sont tels que définis à l'une quelconque des revendications 1, et 5 à 7, sous forme d'un mélange d'isomères optiques selon toutes proportions, ou sous forme enrichie en un isomère optique.

9. Composés (I) selon l'une quelconque des revendications 1 à 2 et 5 à 7 de formule (Ic) : 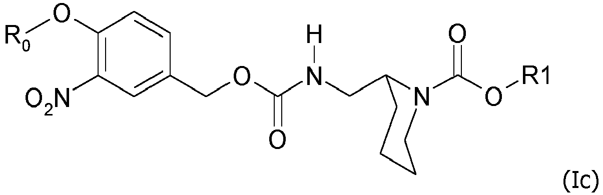 où R0 et R1 sont tels que définis à l'une quelconque des revendications 1 et 5 à 7, sous forme d'un mélange d'isomères optiques selon toutes proportions, ou sous une forme enrichie en un isomère optique.

10. Composés (I) selon l'une quelconque des revendications 1 à 9 pour la mise en œuvre d'une méthode de détection *in vivo,* chez l'homme , d'une glycosidase.

11. Procédé pour détecter *in vitro* ou *ex vivo* la présence d'une glycosidase, comprenant les étapes de :
- mise en contact d'un échantillon suspecté de contenir ladite glycosidase avec un composé (I) selon l'une quelconque des revendications 1 à 9,
- application de conditions appropriées pour permettre la formation d'un précipité fluorescent par clivage de la liaison covalente entre O et R0, suivi d'un clivage de la liaison -C(O)-OR1 conduisant à la libération de HOR1, et
- analyse quantitative ou qualitative dudit précipité fluorescent.

12. Composés de formule (II) : 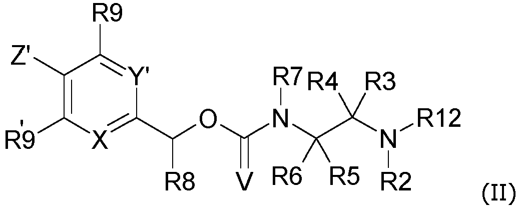 dans laquelle :
- R2, R3, R4, R5, R6, R7, R8, R9, R'9 et V sont tels que définis dans l'une quelconque des revendications 1 à 4,
- R12 représente un atome d'hydrogène, ou un groupe protecteur des fonctions amine,
- X, Y' et Z' sont tels que :
∘ soit X représente CR10, Y' représente CR'10 et Z' représente OR'0,
∘ soit X représente CR10, Y' représente COR'0 et Z' représente R'10,
∘ soit X représente CR10, Y' représente un atome d'azote et Z' représente OR'0,
∘ soit X représente un atome d'azote, Y' représente COR'0 et Z représente R10,
avec R'0 représentant un groupe R0 dont toutes les fonctions alcools sont protégées par un groupe protecteur, et R0, R10 et R'10 étant tels que définis dans la revendication 1,
sous la forme d'un isomère optique selon toutes proportions, ou sous une forme enrichie en un isomère optique.

13. Procédé de préparation d'un composé de formule (I) selon l'une quelconque des revendications 1 à 9 comprenant les étapes suivantes :
- disposer d'un composé (II) selon la revendication 12,
- disposer d'un composé (III) de formule : 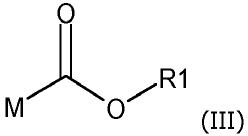 avec R1 tel que défini dans l'une quelconque des revendications 1 et 5 à 6 et M représentant un groupe partant, notamment un atome d'halogène, et en particulier un atome de chlore, un groupe imidazolyle ou un paranitrophénoxy, et de préférence M représentant un para-nitrophenoxyl,
- obtenir le composé (IV) par réaction d'addition dudit composé (II) sur ledit composé (III), ledit composé (IV) ayant pour formule : 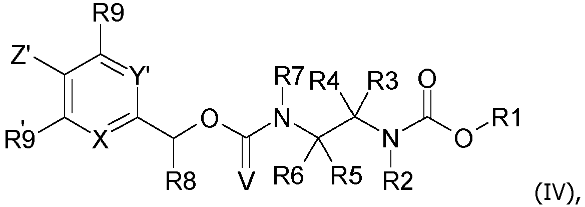 dans lequel R1, R2, R3, R4, R5, R6, R7, R8, R9, R'9, V, X, Y', et Z' sont tels que définis à l'une quelconque des revendications 1 à 7 et 12, et
- déprotéger les fonctions alcools présentes dans le groupe R'0 dudit composés (IV) pour obtenir ledit composé (I).

## Patentansprüche

1. Verbindungen der Formel (I): 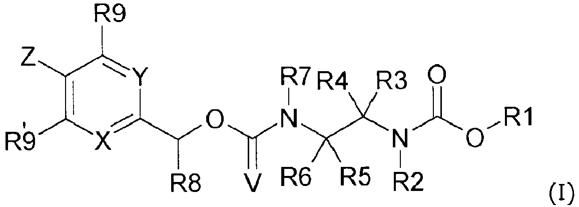 in der:
- R1 wie zum Beispiel HOR1 ist, der nach Spaltung der in Formel (I) vorhandenen -C(O)-OR1-Bindung erhalten wird und zur Klasse der Fluorophore gehört, die zu einem intramolekularen Protonentransfer in einen angeregten Zustand führen, als ESIPT bezeichnet, wobei R1 eine aromatische Gruppe mit -OR1 ist, die der folgenden Formel (A1) entspricht: 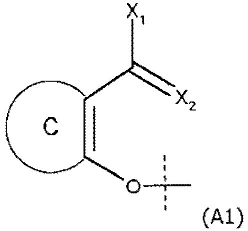
in der:
- entweder X2 ein Sauerstoffatom ist und X1 eine Gruppe -NH₂, -OH, -SH, (C1-C20)Alkyl, (C5-C24)Aryl, -O-(C1-C20)Alkyl, -O-Phenyl, -NH-(C1-C20)Alkyl oder -NH-Phenyl, -S-(C1-C20)Alkyl oder -S-(C5-C24)Aryl ist, wobei die Alkyl- und Phenylgruppen substituiert oder nicht substituiert sein können,
oder X2 für ein Stickstoffatom steht und an X1 gebunden ist, das nun für CH, O, S, N oder NH steht, um ein substituiertes oder nicht substituiertes (C5-C24)Heteroaryl zu bilden, 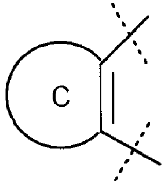 für ein (C5-C24)Aryl oder ein (C5-C24)Heteroaryl steht, das substituiert oder nicht substituiert ist, zum Beispiel ausgewählt aus Phenyl- oder Naphthylgruppen, und: 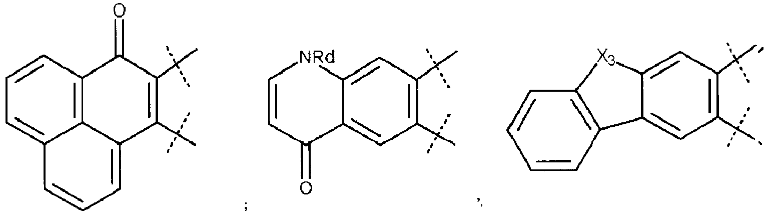 wobei die Gruppen substituiert oder nicht substituiert sein können,
- mit X3, das für S, O oder NRd steht und Rd, das für ein Wasserstoffatom oder eine (C1-C4)Alkylgruppe steht,
- R2, R3 und R4 folgendermaßen definiert sind:
∘ entweder R2 ein (C1-C4)Alkyl ist, R3 ein (C1-C4)Alkyl oder ein Wasserstoffatom ist und R4 ein (C1-C4)Alkyl ist,
∘ oder R3 ein (C1-C4)Alkyl oder ein Wasserstoffatom ist und R2 und R4 miteinander verbunden sind und mit den Kohlenstoff- und Stickstoffatomen, an die sie gebunden sind, einen aliphatischen Heterocyclus bilden, wobei dieser Heterocyclus mit einer hydrosolubilisierenden Gruppe substituiert sein kann,
∘ oder R2 ein (C1-C4)Alkyl ist und R3 und R4 miteinander verbunden sind und mit dem Kohlenstoffatom, an das sie gebunden sind, einen aliphatischen Kohlenstoffring bilden,
- R5 und R6, die identisch oder verschieden sind und unabhängig voneinander für ein Wasserstoffatom, ein (C1-C4)Alkyl oder ein (C5-C10)Aryl stehen,
- R7 ein Wasserstoffatom oder eine Gruppe ist, ausgewählt aus (C1-C4)Alkyl und (C1-C4)Alkoxy,
- R8 für ein Wasserstoffatom oder eine substituierte oder nicht substituierte (C1-C10)Alkylgruppe oder eine Gruppe -D1-D2-D3 steht, wobei:
∘ D1 für eine Triazolyl- oder -CH₂-Triazolylgruppe steht,
∘ D2 für eine (C1-C10)Alkylen-, (C1-C10)Alkenylen- oder (C1-C10)Alkinylengruppe, wobei die Gruppen gegebenenfalls von einem oder mehreren Heteroatomen unterbrochen sind, ausgewählt aus O oder N, eine divalente Glycosylgruppe, eine Gruppe -O-(CHR-CHR'-O)ₙ- oder -N-(CHR-CHR')ₙ- steht, wobei n eine ganze Zahl von 1 bis 20 ist, R und R', die identisch oder verschieden sind, für H oder CH₃ stehen unter der Bedingung, dass R und R' nicht gleichzeitig CH₃, eine Aminosäure oder ein Peptid oder eine Kombination dieser Gruppen sind,
∘ D3 für eine Maleimidocaproyl-Einheit, eine Aminosäure, ein Peptid, eine Folsäure, einen Antikörper oder ein Antikörperfragment steht, gebunden an D2, über eine darin enthaltene Carboxylsäurefunktion, um eine Ester- oder Amidbindung zu bilden,
- R9 und R'9, die identisch oder verschieden sind, für ein Wasserstoffatom oder eine elektronenziehende Gruppe stehen, wie zum Beispiel ein Halogenatom oder eine Gruppe, ausgewählt aus -NO₂, -CN, oder eine Gruppe, ausgewählt aus -NH-C(O)-CH₂-Ab, wobei Ab für einen Antikörper steht,
- V für ein Sauerstoffatom oder ein Schwefelatom steht,
- X, Y und Z zum Beispiel sind:
∘ entweder X für CR10 steht, Y für CR'10 steht und Z für OR0 steht,
∘ oder X für CR10 steht, Y für CORO steht und Z für R'10 steht,
∘ oder X für CR10 steht, Y für ein Stickstoffatom steht und Z für OR0 steht,
∘ oder X für ein Stickstoffatom steht, Y für CORO steht und Z für R10 steht,
wobei:
∘ R0 für eine Glycosylgruppe steht, die über ihren anomeren Kohlenstoff an den Rest des Moleküls der Formel (I) gebunden ist, und
o R10 und R'10, die identisch oder verschieden sind, für ein Wasserstoffatom oder eine Elektronendonatorgruppe stehen, wie ein (C1-C20)Alkyl, ein (C5-C24)Aryl oder ein (C 1-C20)Alkoxy,
in Form einer Mischung von optischen Isomere in beliebigen Verhältnissen oder in einer Form, die mit einem optischen Isomer angereichert ist.

2. Verbindungen (I) gemäß Anspruch 1, **dadurch gekennzeichnet, dass** R3 ein Wasserstoffatom oder eine (C1-C4)Alkylgruppe, bevorzugt ein Wasserstoffatom, ist, und R2 und R4 miteinander verbunden sind und eine -(CH₂)ₘ-Kette mit m = 3, 4 oder 5 bilden.

3. Verbindungen (I) gemäß Anspruch 1, **dadurch gekennzeichnet, dass** R3 ein Wasserstoffatom oder eine (C1-C4)Alkylgruppe, bevorzugt ein Wasserstoffatom, ist, und R2 und R4 miteinander verbunden sind und eine -CH₂CH₂-NR11-CH₂- Kette in Richtung von R2 nach R4 bilden, wobei R11 für ein Wasserstoffatom oder -(L)n-GP steht mit n gleich 0 oder 1, L ein Bindungsarm und GP eine hydrosolubilisierende Gruppe ist.

4. Verbindungen (I) gemäß Anspruch 1, **dadurch gekennzeichnet, dass** R2, R3 und R4, die identisch oder verschieden sind, für eine (C1-C4)Alkylgruppe, zum Beispiel Methyl oder Ethyl, stehen.

5. Verbindungen (I) gemäß einem der Ansprüche 1 bis 4, **dadurch gekennzeichnet, dass** -OR1 vom Typ Phenoxy ist, und einer der folgenden Strukturen (A2) oder (A3) entspricht: 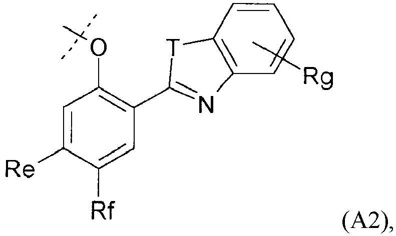 in der:
∘ T-NH-C(O)-, -S-, -O-, -NH-, -N((C1-C20)Alkyl)- oder -N((C5-C24)Aryl)- ist,
∘ Re ein Wasserstoffatom oder ein elektronenziehender kohlenstoffhaltiger Substituent wie -CN oder -COORh ist, mit Rh, der für eine (C1-C4)Alkylgruppe steht, oder Re - CONRiRj ist, wobei Ri und Rj, die identisch oder verschieden sind, für ein Wasserstoffatom oder eine (C1-C4)Alkylgruppe stehen oder Re -CF₃ ist oder eine 2-Oxazolyl-, 2-Thiazolyl-, 2-Imidazolyl-, 2-Benzoimidazolyl, 4-Pyrimidinon-2-yl- oder Chinazolinon-2-yl-Gruppe ist,
∘ Rf ein Wasserstoffatom, ein Chlor-, Brom-, Iod- oder Fluoratom, -OH, -NH₂, NRkRl, -NHRk oder -ORk ist, mit Rk und Rl, die identisch oder verschieden sind und jeweils unabhängig voneinander für eine (C1-C4)Alkylgruppe stehen,
∘ oder Re und Rf miteinander verbunden sind, um eine Kohlenwasserstoffkette, umfassend 4 oder 5 Kettenglieder, die gesättigt oder ungesättigt, substituiert oder nicht substituiert, gegebenenfalls von einem oder mehreren Heteroatomen, ausgewählt aus N, S und O, unterbrochen sind,
o Rg ein Wasserstoffatom, Br, Cl, I oder F ist, 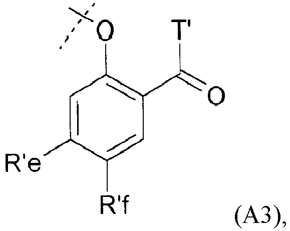 in der:
∘ T' -NH₂, -OH, eine (C5-C24)Arylgruppe, eine (C1-C4)Alkylgruppe, -SH, -NHR'g, - OR'g, -NR'gRh' oder -SR'g ist, wobei R'g und R'h, die identisch oder verschieden sind, für eine (C1-C4)Alkyl- oder Arylgruppe stehen,
∘ R'e ein Wasserstoffatom oder ein kohlenstoffhaltiger elektronenziehender Substituent ist, wie -CN oder -COOR'i ist, mit R'i, der für eine (C1-C4)Alkylgruppe steht, oder R'e -CONR'jR'k ist, mit R'j und R'k, die identisch oder verschieden sind und für ein Wasserstoffatom oder eine (C1-C4)Alkylgruppe stehen oder R'e -CF₃ oder eine 2-Oxazolyl-, 2-Thiazolyl-, 2-Imidazolyl-, 2-Benzoimidazolyl, 4-Pyrimidinon-2-yl- oder Chinazolinon-2-yl-Gruppe ist,
o R'f ein Wasserstoffatom, ein Chlor-, Brom-, Iod- oder Fluoratom, -OH, -NH₂, NR'lR'm, oder -OR'l ist, mit R'l und R'm, die identisch oder verschieden sind und für eine (C1-C4)Alkylgruppe stehen,
∘ oder R'e und R'f miteinander verbunden sind, um eine Kohlenwasserstoffkette, umfassend 4 oder 5 Kettenglieder, die gesättigt oder ungesättigt, substituiert oder nicht substituiert, gegebenenfalls von einem oder mehreren Heteroatomen, ausgewählt aus N, S und O, unterbrochen sind.

6. Verbindungen (I) gemäß einem der Ansprüche 1 bis 5, **dadurch gekennzeichnet, dass** R1 eine aromatische Gruppe mit -OCR1 ist, die der folgenden Formel (A5) entspricht:

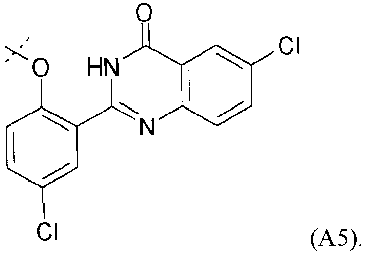

7. Verbindungen (I) gemäß einem der Ansprüche 1 bis 6, **dadurch gekennzeichnet, dass** R0 eine monoglycosylierte Gruppe ist, die über ihren anomeren Kohlenstoff gebunden ist und aus Galactosyl, Glucosyl, Mannosyl, Gulosyl, Allosyl, Altrosyl, Idosyl, Talosyl, Fucosyl, Fructosyl, Arabinosyl, Lyxosyl, Ribosyl, Xylosyl, Glucuronyl, und N-Acetylhexosaminyl oder einer polyglycosylierten Gruppe, gebildet aus mehreren identischen oder verschiedenen dieser monoglycosylierten Gruppen, ausgewählt ist.

8. Verbindungen (I) gemäß einem der Ansprüche 1 bis 7 der Formel (Ib): 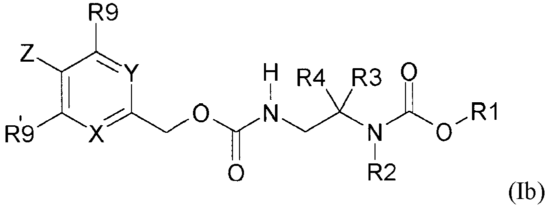 wo R1, R2, R3, R4, R9, R'9, X, Y und Z wie nach einem der Ansprüche 1 und 5 bis 7 definiert sind in Form einer Mischung von optischen Isomeren in beliebigen Verhältnissen oder in einer Form, die mit einem optischen Isomer angereichert ist.

9. Verbindungen (I) gemäß einem der Ansprüche 1 bis 2 und 5 bis 7 der Formel (Ic): 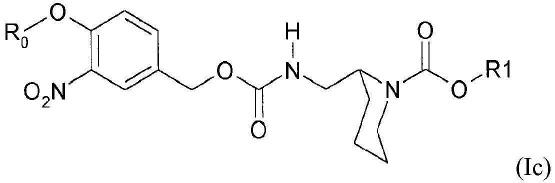 wo R0 und R1 wie nach einem der Ansprüche 1 und 5 bis 7 definiert sind, in Form einer Mischung von optischen Isomeren in beliebigen Verhältnissen oder in Form eines angereicherten optischen Isomers.

10. Verbindungen (I) gemäß einem der Ansprüche 1 bis 9 zur Durchführung eines Verfahrens zur *in vivo* Detektion einer Glycosidase beim Menschen.

11. Verfahren zur *in vitro* oder *ex vivo* Detektion des Vorhandenseins einer Glycosidase, umfassend die Schritte:
- Inkontaktbringen einer Probe, die die Glycosidase vermutlich enthält, mit einer Verbindung (I) gemäß einem der Ansprüche 1 bis 9,
- Anwendung geeigneter Bedingungen, um die Bildung eines fluoreszierenden Präzipitats mittels Spaltung der kovalenten Bindung zwischen O und R0 und anschließender Spaltung der Bindung -C(O)-OR1, die zur Freisetzung von HOR1 führt, zu erlauben, und
- qualitative oder quantitative Analyse des fluoreszierenden Präzipitats.

12. Verbindungen der Formel (II): 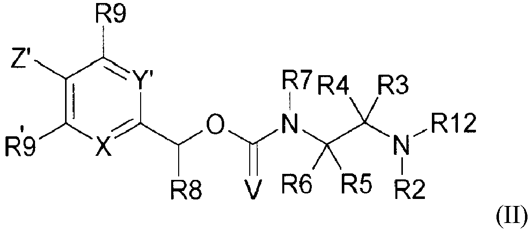 in der
- R2, R3, R4, R5, R6, R7, R8, R9, R'9 und V wie in einem der Ansprüche 1 bis 4 definiert sind,
- R12 für ein Wasserstoffatom oder eine Schutzgruppe der Aminfunktionen steht,
- X, Y' und Z' zum Beispiel sind:
∘ entweder X für CR10 steht, Y' für CR'10 steht und Z' für OR'0 steht,
∘ oder X für CR10 steht, Y' für COR'0 steht und Z' für R'10 steht,
∘ oder X für CR10 steht, Y' für ein Stickstoffatom steht und Z' für OR'0 steht,
∘ oder X für ein Stickstoffatom steht, Y' für COR'0 steht und Z' für R10 steht,
wobei R'0 für eine Gruppe R0 steht, deren sämtliche Alkoholfunktionen mit einer Schutzgruppe geschützt sind, und R0, R10 und R'10 wie in Anspruch 1 definiert sind,
in Form eines optischen Isomers in beliebigen Verhältnissen oder in einer Form, die mit einem optischen Isomer angereichert ist.

13. Verfahren zur Herstellung einer Verbindung der Formel (I) gemäß einem der Ansprüche 1 bis 9, umfassend die folgenden Schritte:
- Bereitstellen einer Verbindung (II) gemäß Anspruch 12,
- Bereitstellen einer Verbindung (III) der Formel: 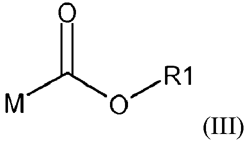 wobei R1 wie in einem der Ansprüche 1 und 5 bis 6 definiert ist und M für eine Abgangsgruppe, insbesondere ein Halogenatom und vor allem ein Chloratom, eine Imidazolyl- oder ein para-Nitrophenoxy-Gruppe steht, und vorzugsweise für ein para-Nitrophenoxyl steht,
- Erhalt der Verbindung (IV) durch Additionsreaktion der Verbindung (II) mit der Verbindung (III), wobei die Verbindung (IV) die Formel besitzt: 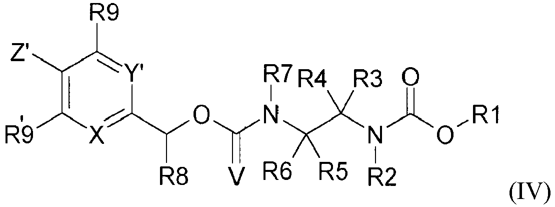 in der R1, R2, R3, R4, R5, R6, R7, R8, R9, R'9, V, X, Y' und Z' wie nach einem der Ansprüche 1 bis 7 und 12 definiert sind, und
- Entschützen der Alkoholfunktionen in der Gruppe R'0 der Verbindungen (IV), um die Verbindung (I) zu erhalten.

## Claims

1. Compounds of formula (I): 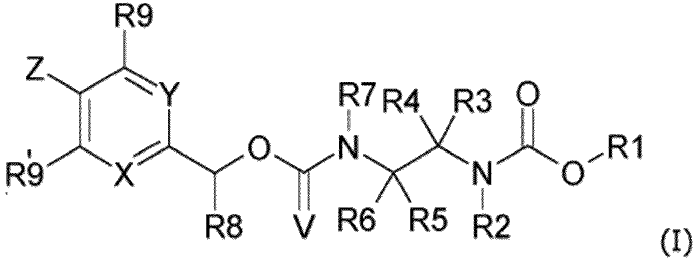 wherein:
- R1 is such that HOR1, obtained after cleaving the -C(O)-OR1 bond present in formula (I), belongs to the class of fluorophores leading to an excited state intramolecular proton transfer, referred to as ESIPT, R1 being an aromatic group with -OR1 which has the formula (A1): 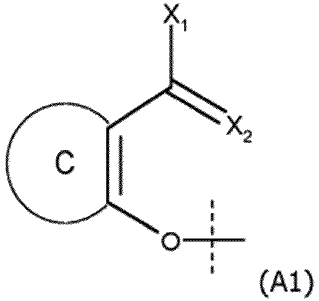 wherein:
- either X2 is an oxygen atom and X1 is a -NH₂, -OH, -SH, (C1-C20) alkyl, (C5-C24) aryl, -O-(C1-C20) alkyl, -O-phenyl, -NH-(C1-C20) alkyl or -NH-phenyl, -S-(C1-C20) alkyl or -S-(C5-C24) aryl group, said alkyl and phenyl groups being substituted or unsubstituted,
or X2 represents a nitrogen atom and is bonded to X1 which then represents CH, O, S, N or NH in order to form a substituted or unsubstituted (C5-C24) heteroaryl, 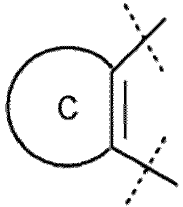 represents a (C5-C24) aryl or a (C5-C24) heteroaryl, substituted or unsubstituted, for example chosen among the phenyl and naphthyl groups, and: 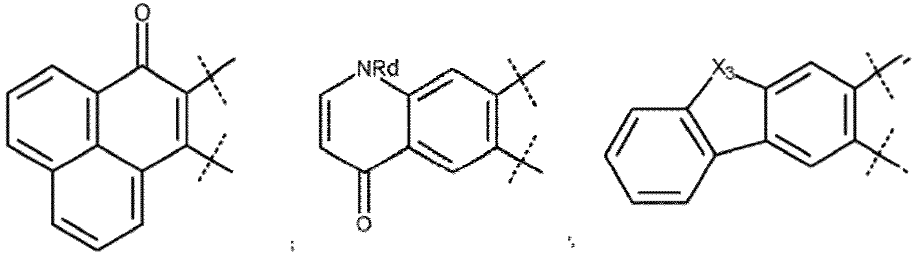 said groups being able to be substituted or unsubstituted,
- with X3 which represents S, O or NRd and Rd which represents a hydrogen atom or a (C1-C4) alkyl group,
- R2, R3 and R4 are defined as follows:
o either R2 is a (C1-C4) alkyl, R3 is a (C1-C4) alkyl or a hydrogen atom, and R4 is a (C1-C4) alkyl,
o or R3 is a (C1-C4) alkyl or a hydrogen atom and R2 and R4 are bonded to one another and form with the carbon and nitrogen atoms to which they are bonded, an aliphatic heterocycle, this heterocycle being able to be substituted by a group conferring solubility in water,
o or R2 is a (C1-C4) alkyl and R3 and R4 are bonded to one another and form an aliphatic carbocycle with the carbon atom to which they are bonded.
- R5 and R6 are identical or different and represent, independently from one another, a hydrogen atom, a (C1-C4) alkyl, or a (C5-C10) aryl,
- R7 is a hydrogen atom, or a group chosen among the (C1-C4) alkyl and (C1-C4) alkoxy,
- R8 represents a hydrogen atom, or a substituted or unsubstituted (C1-C10) alkyl group, or a -D1-D2-D3 group with:
∘ D1 representing a triazolyl or -CH2-triazolyl group,
∘ D2 representing a (C1-C10) alkylene, (C1-C10) alkenylene, or (C1-C10) alkynylene group, said groups being optionally interrupted by one or more heteroatoms chosen from O or N, a divalent glycosyl group, a -O-(CHR-CHR'-O)ₙ-or -N-(CHR-CHR'-O)ₙ- group, n being an integer ranging from 1 to 20, R and R', identical or different, representing H or CH₃ provided that R and R' are not simultaneously CH₃, an amino acid or a peptide, or a combination of these groups,
∘ D3 representing a maleimidocaproyl unit, amino acid, peptide, folic acid, antibody or antibody fragment bonded to D2, by a carboxylic acid function that it comprises, so as to form an ester or amide bond,
- R9 and R'9, identical or different, represent a hydrogen atom, or an electron-withdrawing group, such as a halogen atom, or a group chosen among -NO₂, -CN, or a group chosen among -NH-C(O)-CH₂Ab, with Ab representing an antibody
- V represents an oxygen atom or a sulfur atom,
- X, Y and Z are such that:
∘ either X represents CR10, Y represents CR'10 and Z represents OR0,
∘ or X represents CR10, Y represents CORO and Z represents R'10,
∘ or X represents CR10, Y represents a nitrogen atom and Z represents OR0,
∘ or X represents a nitrogen atom, Y represents CORO and Z represents R10 with:
∘ R0 representing a glycosyl group bonded by its anomeric carbon to the remainder of the molecule of formula (I), and
∘ R10 and R'10, identical or different, representing a hydrogen atom or an electron-donating group, such as a (C1-C20) alkyl, a (C5-C24) aryl, or a (C1-C20) alkoxy,
in the form of a mixture of optical isomers in any proportion, or in a form enriched in one optical isomer.

2. The compounds (I) according to claim 1, **characterised in that** R3 is a hydrogen atom or a (C1-C4) alkyl group, preferably a hydrogen atom, and R2 and R4 are bonded to one another and form a -(CH₂)ₘ- sequence with m = 3, 4 or 5.

3. The compounds (I) according to claim 1, **characterised in that** R3 is a hydrogen atom or a (C1-C4) alkyl group, preferably a hydrogen atom, and R2 and R4 are bonded to one another and form a sequence -CH₂CH₂₋NR11-CH₂⁻ in the direction from R2 to R4, R11 representing a hydrogen atom or -(L)n-GP with n which is equal to 0 or 1, L a connecting arm and GP a group conferring water-solubility

4. The compounds (I) according to claim 1, **characterised in that** R2, R3 and R4, identical or different, represent a group (C1-C4) alkyl, for example methyl or ethyl.

5. The compounds (I) according to any one of claims 1 to 4, characterised in that-OR1 is of the phenoxy type, and corresponds to one of the following structures (A2) or (A3): 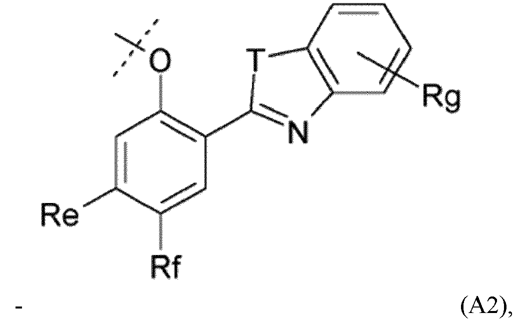 wherein:
∘ T is -NH-C(O)-, -S-, -O-, -NH-, -N((C1-C20) alkyl)- or -N((C5-C24) aryl)-
∘ Re is a hydrogen atom or an electron-withdrawing carbon substituent such as -CN or -COORh, with Rh which represents a (C1-C4) alkyl group, or else Re is -CONRiRj, with Ri and Rj, identical or different, which represent a hydrogen atom or a (C1-C4) alkyl group, or else Re is -CF₃, or a 2-oxazolyl, 2-thiazolyl, 2-imidazolyl, 2-benzoimidazolyl, 4-pyrimidinon-2-yl or quinazolinon-2-yl group,
∘ Rf is a hydrogen, chlorine, bromine, iodine or fluorine atom, - OH, -NH₂, - NRkRl, -NHRk or -ORk, with Rk and Rl identical or different which each independently represent a (C1-C4) alkyl group,
∘ or else Re and Rf are bonded to one another in order to form a saturated or unsaturated, substituted or unsubstituted, hydrocarbon chain comprising 4 or 5 chain units, optionally interrupted by one or more heteroatoms chosen from N, S and O,
∘ Rg is a hydrogen, Br, Cl, I or F atom 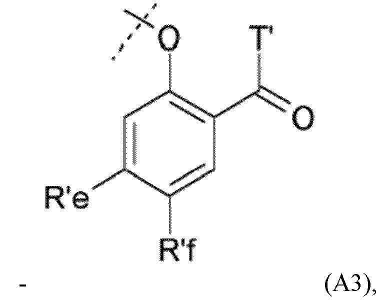 wherein:
∘ T' is -NH₂, -OH, a (C5-C24) aryl group, a (C1-C4) alkyl group, -SH, - NHR'g, -OR'g, -NR'gRh' or -SR'g, R'g and Rh', identical or different, representing a (C1-C4) alkyl or aryl group,
∘ R'e is a hydrogen atom or an electron-withdrawing carbon substituent such as -CN, or -COOR'i, with R'i which represents a (C1-C4) alkyl group, or R'e is - CONR'jR'k, with R'j and R'k, identical or different, which represent a hydrogen atom or a (C1- C4) alkyl group, or else R'e is -CF₃, or a 2-oxazolyl, 2-thiazolyl, 2-imidazolyl, 2-benzoimidazolyl, 4-pyrimidinon-2-yl, or quinazolinon-2-yl group,
∘ R'f is a hydrogen, chlorine, bromine, iodine or fluorine atom, - OH, -NH₂, - NR'IR'm or -OR'l, with R'l and R'm, identical or different, which represent a (C1-C4) alkyl group,
∘ or else R'e and R'f are bonded to one another in order to form a saturated or unsaturated, substituted or unsubstituted, hydrocarbon chain comprising 4 or 5 chain units, optionally interrupted by one or more heteroatoms chosen from N, S and O.

6. The compounds (I) according to any one of claims 1 to 5, **characterised in that** R1 is an aromatic group with -OR1 which has the following formula (A5):

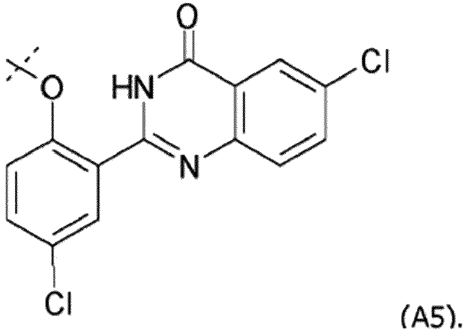

7. The compounds (I) according to any one of claims 1 to 6, **characterised in that** R0 is a monoglycosylated group bonded by its anomeric carbon chosen among the galactosyl, glucosyl, mannosyl, gulosyl, allosyl, altrosyl, idosyl, talosyl, fucosyl, fructosyl, arabinosyl, lyxosyl, ribosyl, xylosyl, glucuronyl and N-acetyl-hexosaminyl groups or a polyglycosylated group consisting of a plurality of these monoglycosylated groups, identical or different.

8. The compounds (I) according to any one of claims 1 to 7 of formula (lb): 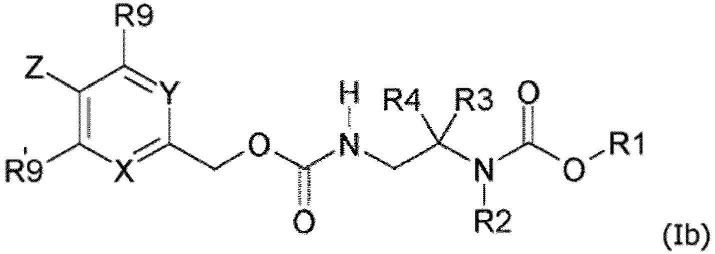 where R1, R2, R3, R4, R9, R'9, X, Y and Z are as defined in any one of claims 1 and 5 to 7, in the form of a mixture of optical isomers in any proportion, or in a form enriched in one optical isomer.

9. The compounds (I) according to any one of claims 1 to 2 and 5 to 7 of formula (Ic): 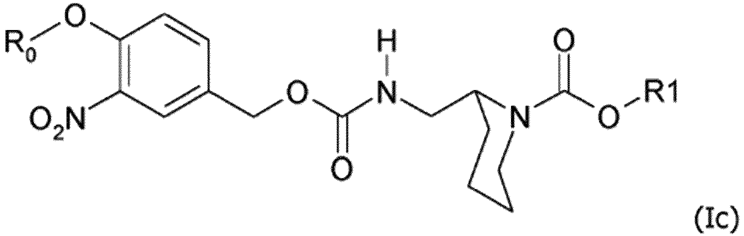 where R0 and R1 are as defined in any one of claims 1 and 5 to 7, in the form of a mixture of optical isomers in any proportion, or in a form enriched in one optical isomer.

10. The compounds (I) according to any one of claims 1 to 9 for implementing a method for *in vivo* detection of a glycosidase in humans.

11. Process for *in vitro* or *ex vivo* detection of the presence of a glycosidase, comprising the steps of:
- placing a sample that is suspected to contain said glycosidase in contact with a compound (I) according to any one of claims 1 to 9,
- applying appropriate conditions for enabling the formation of a fluorescent precipitate by cleavage of the covalent bond between O and R0, followed by cleaving of the -C(O)-OR1 bond leading to the release of HOR1, and
- quantitative or qualitative analysis of said fluorescent precipitate.

12. Compounds of formula (II): 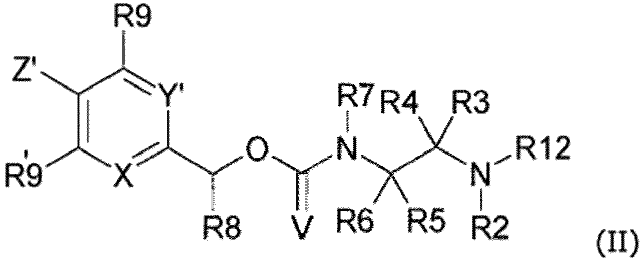 wherein:
- R2, R3, R4, R5, R6, R7, R8, R9, R'9 and V are as defined in any one of claims 1 to 4,
- R12 represents a hydrogen atom, or a protecting group for amine functions, - X, Y' and Z' are such that:
oeither X represents CR10, Y' represents CR'10 and Z' represents OR'0,
oor X represents CR10, Y' represents COR'0 and Z' represents R'10,
oor X represents CR10, Y' represents a nitrogen atom and Z' represents OR'0,
oor X represents a nitrogen atom, Y' represents COR'0 and Z represents R10,
with R'0 representing a group R0 for which all of the alcohol functions are protected by a protecting group, and R0, R10 and R'10 being as defined in claim 1,
in the form of an optical isomer in any proportion, or in a form enriched in one optical isomer.

13. Process for preparing a compound of formula (I) according to any one of claims 1 to 9 comprising the following steps:
- providing a compound (II) according to claim 12,
- providing a compound (III) of formula: 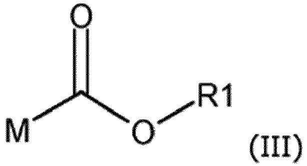 with R1 as defined in any one of claims 1 and 5 to 6 and M representing a leaving group, in particular a halogen atom, and in particular a chlorine atom, an imidazolyl or a para-nitrophenoxy group, and M preferably representing a para-nitrophenoxyl,
- obtaining the compound (IV) by addition reaction of said compound (II) on said compound (III), said compound (IV) having the formula: 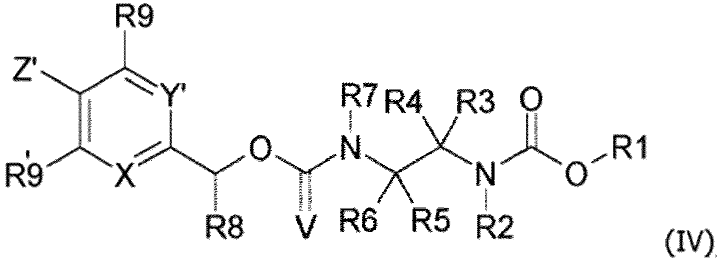 wherein R1, R2, R3, R4, RS, R6, R7, R8, R9, R'9, V, X, Y', and Z' are as defined in any one of claims 1 to 7 and 12, and
- deprotecting the alcohol functions present in the R'0 group of said compounds (IV) in order to obtain said compound (I).
